(19) Europäisches Patentamt
European Patent Office
Office européen des brevets

(11) **EP 1 633 206 B1**

(12) **EUROPÄISCHE PATENTSCHRIFT**


(45) Veröffentlichungstag und Bekanntmachung des Hinweises auf die Patenterteilung:
**15.08.2007 Patentblatt 2007/33**

(21) Anmeldenummer: **04741635.9**

(22) Anmeldetag: **24.05.2004**

(51) Int Cl.:
***A23L 1/272*** *(2006.01)*

(86) Internationale Anmeldenummer:
**PCT/EP2004/050896**

(87) Internationale Veröffentlichungsnummer:
**WO 2004/105736 (09.12.2004 Gazette 2004/50)**


(54) **VERWENDUNG VON DIPHENYLMETHAN-DERIVATEN ALS TYROSINASE-INHIBITOREN**

USE OF DIPHENYLMETHANE DERIVATIVES AS TYROSINASE INHIBITORS

UTILISATION DE DERIVES DE DIPHENYLMETHANE COMME INHIBITEURS DE LA TYROSINASE


(84) Benannte Vertragsstaaten:
**AT BE BG CH CY CZ DE DK EE ES FI FR GB GR HU IE IT LI LU MC NL PL PT RO SE SI SK TR**

(30) Priorität: **30.05.2003 DE 10324566**

(43) Veröffentlichungstag der Anmeldung:
**15.03.2006 Patentblatt 2006/11**



(73) Patentinhaber: **Symrise GmbH & Co. KG**
**37603 Holzminden (DE)**

(72) Erfinder:
- **SCHMAUS, Gerhard**
  **37671 Höxter-Bosseborn (DE)**
- **HERRMANN, Martina**
  **37574 Einbeck (DE)**
- **JOPPE, Holger**
  **37586 Dassel (DE)**
- **VIELHABER, Gabriele**
  **37603 Holzminden (DE)**

(74) Vertreter: **Eisenführ, Speiser & Partner**
**Patentanwälte Rechtsanwälte**
**Postfach 10 60 78**
**28060 Bremen (DE)**



(56) Entgegenhaltungen:
**EP-A- 0 423 470**
**EP-A- 0 811 595**
**WO-A-00/56279**
**WO-A-03/049713**
**US-A- 5 202 141**
**US-A- 5 399 785**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).


EP 1 633 206 B1

## Beschreibung

Die vorliegende Erfindung betrifft die Verwendungen von Diphenylmethan-Derivaten der nachfolgenden Formel 1 als Tyrosinase-Inhibitioren bzw. zur Herstellung eines Mittels zur Hautaufhellung und/oder zur Bekämpfung von Altersflecken,

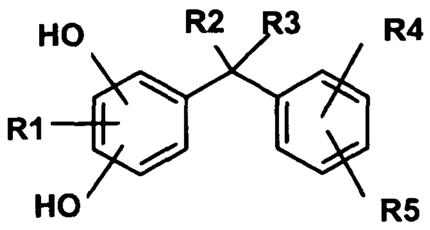

**1**

wobei gilt:

R1 ist

- Wasserstoff,
- Methyl,
- geradkettiges oder verzweigtes, gesättigtes oder ungesättigtes Alkyl mit 2-4 C-Atomen
- OH oder
- Halogen,

R2 ist

- Wasserstoff,
- Methyl oder
- geradkettiges oder verzweigtes, gesättigtes oder ungesättigtes Alkyl mit 2-5 C-Atomen,

R3 ist

- Methyl oder
- geradkettiges oder verzweigtes, gesättigtes oder ungesättigtes Alkyl mit 2-5 C-Atomen,

und

R4 und R5 sind unabhängig voneinander

- Wasserstoff,
- Methyl,
- geradkettiges oder verzweigtes, gesättigtes oder ungesättigtes Alkyl mit 2-5 C-Atomen

- OH oder
- Halogen.

Die Substituenten OH, R1, R4 und R5 können dabei jeweils (wie zeichnerisch angezeigt) eine beliebige Position am jeweiligen aromatischen Ring einnehmen (ortho, meta oder para zu der Brücke zwischen den Ringen).

Im Bereich der kosmetischen Industrie besteht ein zunehmender Bedarf an Mitteln zur Haut-und Haaraufhellung sowie an Mitteln zur Bekämpfung von Altersflecken. Kosmetika zur Haut- und Haaraufhellung und zur Bekämpfung von Altersflecken spielen dabei vor allem in asiatischen Ländern mit dunkelhäutiger/haariger Bevölkerung eine große Rolle, aber auch im mitteleuropäischen Raum und in den USA gewinnen Mittel für derartige kosmetische Behandlungen zunehmend an Bedeutung.

Die Haut- und Haarfarbe der Menschen wird im wesentlichen über die Melanocytenzahl, über die Melaninkonzentration und die Intensität der Melaninbiosynthese bestimmt, wobei zum einen intrinsische Faktoren wie die genetische Ausstattung eines Individuums und zum anderen extrinsische Faktoren wie insbesondere die Intensität und Häufigkeit der UV-Exposition einen signifikanten Einfluss auf die Haut- und Haarfarbe ausüben.

Hautaufhellende Wirkstoffe greifen üblicherweise in den Melaninmetabolismus bzw. -katabolismus ein. Die in der Regel braun bis schwarz gefärbten Melanin-Pigmente werden in den Melanozyten der Haut gebildet, in die Keratinozyten übertragenHau und verursachen die Färbung der Haut oder der Haare. Die braun-schwarzen Eumelanine werden in Säugetieren hauptsächlich aus hydroxysubstituierten aromatischen Aminosäuren wie L-Tyrosin und L-DOPA, die gelben bis roten Phäomelanine zusätzlich aus schwefelhaltigen Molekülen gebildet (Cosmetics & Toiletries 1996, 111 (5), 43-51). Ausgehend von L-Tyrosin wird durch das kupferhaltige Schlüsselenzym Tyrosinase L-3,4-Dihydroxyphenylalanin (L-DOPA) gebildet, welches wiederum durch Tyrosinase zu Dopachrom umgewandelt wird. Letzteres wird über mehrere, durch verschiedene Enzyme katalysierte Schritte zu Melanin oxidiert.

Hautaufhellende Mittel werden aus verschiedenen Gründen gebraucht: Sind die Melanin-bildenden Melanozyten in der menschlichen Haut aus irgendeinem Grund nicht gleichmäßig verteilt, kommt es zu Pigmentflecken, die entweder heller oder dunkler als die umgebenden Hautareale sind. Um dieses Problem zu beheben, werden Aufhellungsmittel eingesetzt, die solche Pigmentflecken zumindest teilweise auszugleichen helfen. Zudem besteht für viele Menschen das Bedürfnis, ihre natürlicherweise dunkle Hautfarbe aufzuhellen oder der Hautpigmentierung vorzubeugen. Dafür sind sehr sichere und wirkungsvolle Haut- und Haaraufhellungsmittel notwendig. Viele Haut-und Haaraufhellungsmittel enthalten mehr oder minder starke Tyrosinase-Inhibitoren. Damit wird jedoch nur ein möglicher Weg der Haut- und Haaraufhellung beschritten.

Gelegentlich werden auch UV-absorbierende Substanzen zum Schutz gegen die durch UV-Licht induzierte Zunahme der Hautpigmentierung eingesetzt. Dies ist jedoch ein rein physikalisch bedingter Effekt und unterscheidet sich damit von der biologischen Wirkung von hautaufhellenden Mitteln auf die zelluläre Melanin-Bildung, die auch in Abwesenheit von UV-Licht nachweisbar ist. Durch UV-Filter kann nämlich nur die UV-induzierte Hautbräunung verhindert werden, wohingegen mit biologisch wirksamen Hautaufhellern, die in die Melaninbiosynthese eingreifen, auch eine Aufhellung der Haut hervorgerufen werden kann.

In handelsüblichen Haut- und Haaraufhellungsmitteln werden insbesondere Hydrochinon, Hydrochinonderivate wie z.B. Arbutin, Vitamin C. Derivate der Ascorbinsäure wie z.B. Ascorbylpalmitat, Kojisäure und Derivate der Kojisäure wie z.B. Kojisäuredipalmitat verwendet.

Eines der am häufigsten verwendeten Haut- und Haaraufhellungsmittel ist Hydrochinon. Die Substanz hat jedoch einen zytotoxischen Effekt auf Melanozyten und wirkt irritierend auf die Haut. Daher sind solche Präparate z.B. in Europa, Japan und Südafrika für kosmetische Anwendungen nicht mehr zulässig. Zudem ist Hydrochinon sehr oxidationsempfindlich und nur schwierig in kosmetischen Formulierungen zu stabilisieren.

Vitamin C und Ascorbinsäurederivate haben nur eine unzureichende Wirkung auf der Haut. Sie wirken zudem nicht direkt als Tyrosinase-Hemmstoffe, sondern reduzieren die farbigen Zwischenstufen der Melaninbiosynthese.

Kojisäure (5-Hydroxy-2-hydroxymethyl-4-pyranon) ist ein Tyrosinase-Inhibitor, der über eine Chelatisierung der Kupferatome des Enzyms dessen katalytische Wirkung hemmt; sie wird in kommerziellen Haut- und Haaraufhellungsmitteln eingesetzt, besitzt jedoch ein hohes sensibilisierendes Potential und verursacht Kontaktallergien.

US 5,399,785 offenbart Tyrosinase-Aktivitätsinhibitoren, welche in der Gesichtskosmetik und als Melanin-Inhibitoren verwendet werden können. Inhibitoren der Strukturformel III stellen Resorcinol-Derivate dar.

WO 00/56279 offenbart die Verwendung von Resorcinol-Derivaten als Hautaufhellungsmittel.

EP 0 811 595 A1 offenbart Hydrochalcon-Derivate, welche sehr wirksam sind in der Verhinderung der Pigmentablagerung sowie der D-Pigmentierung der Haut und welche weiterhin sicher in der Anwendung auf der Haut und lagerungsstabil sind.

Bei der Suche nach neuen haut- und haaraufhellend wirksamen und/oder gegen Altersflecken wirksamen Mitteln ist man dementsprechend ganz allgemein bestrebt Substanzen zu finden, die in möglichst geringer Konzentration das Enzym Tyrosinase inhibieren, wobei weiterhin zu beachten ist, dass diese in kosmetischen und/oder pharmazeutischen Produkten verwendeten Substanzen neben einer hohen Wirksamkeit bei möglichst niedrigen Konzentrationen auch noch

- toxikologisch unbedenklich,
- gut hautverträglich, insbesondere nicht sensibilisierend und nicht irritierend,
- stabil (insbesondere in den üblichen kosmetischen und/oder pharmazeutischen Formulierungen),
- vorzugsweise geruchlos und
- preiswert herstellbar (d.h. unter Einsatz von Standardverfahren und/oder ausgehend von Standardprekursoren)

sein müssen.

Die Suche nach geeigneten (Wirk-)substanzen, die eine oder mehrere der genannten Eigenschaften in ausreichendem Maße besitzen, ist dem Fachmann dadurch erschwert, dass keine klare Abhängigkeit zwischen der chemischen Struktur einer Substanz einerseits und ihrer biologischen Aktivität sowie ihrer Stabilität andererseits besteht. Des Weiteren gibt es keinen vorhersehbaren Zusammenhang zwischen der hautaufhellenden Wirkung, der toxikologischen Unbedenklichkeit, der Hautverträglichkeit und/oder der Stabilität potentieller Wirkstoffe. Eine besondere Voraussetzung für den Einsatz einer Wirksubstanz in der Praxis ist darüberhinaus ihre Stabilität gegenüber chemischen Substanzen, welche als Begleitstoffe in Kosmetika üblicherweise Anwendung finden und gegenüber Licht.

Es war daher (im Einklang mit den allgemeinen Erfordernissen, s.o.) die primäre Aufgabe der vorliegenden Erfindung, einen Wirkstoff anzugeben, welcher (a) eine gute hautaufhellende Wirkung besitzt (d.h. zum Beispiel eine starke Tyrosinaso-inhibierende Wirkung in spezifischen zellfreien oder zellulären in-vitro Test-Systemen, wobei zellulären in vitro-Testsystemen aufgrund der besseren Übertragbarkeit auf die humane in-vivo-Situation der Vorzug gegeben wurde), (b) in hochreiner Form herstellbar ist, (c) dermatologisch und toxikologisch unbedenkbar ist und (d) darüber hinaus gegenüber den Einflüssen von Licht eine gute Stabilität zeigt Eigene Untersuchungen zeigten nun, dass Diphenylmethan-Derivate der Formel 1 diese Aufgaben lösen und somit bevorzugt als Tyrosinase-inhibierende Mittel zum Einsatz gelangen können.

Erfindungsgemäß einsetzbare Diphenytmethan-Deftvate wie z.B. das im folgenden näher beschriebene Styrylresorcinol (Formel 4; CARN:85-27-8; 4-(1-Phenylethyl)-1,3-dihydroxybenzol), lassen sich dabei problemlos nach literaturbekannten Verfahren herstellen. Für die Durchführung von Wirksamkeitsstudien wurden die Diphenytmethan-Denvate der Formel 1 durch Friedel-Crafts-Alkylierung gemäß literaturbekannten Verfahren, wie in T. Yamamura et al. (Bull. Chem. Soc. Jpn. Vol. 68, S.2955-2960; 1995) beschrieben, hergestellt.

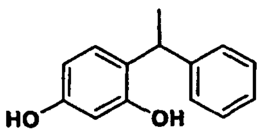

4

Eigene Untersuchungen zeigten nun, dass Diphenylmethan-Derivate der Formel 1, und hier insbesondere das Styrylresorcinol der Formel 4 eine stärkere Tyrosinase-inhibierende Wirksamkeit aufweisen als die u.a. in der Lebensmittelindustrie als Bräunungsinhibitor eingesetzte Verbindung Hexylresorcinol (siehe unten Beispiel 1, Tabelle 2: Vergleich Styrylresorcinol (Formel 4) und 4-Hexylresorcinol: CARN: 136-77-6). Besonders überraschend war darüber hinaus die Tatsache, dass Verbindungen der Formel 1, und hier insbesondere Styrylresorcinol (Formel 4) gegenüber dem bekannten haut- und haaraufhellenden Wirkstoff Kojisäure eine stärkere Tyrosinase-inhibierende Wirksamkeit besitzen, weshalb sie in besonders niedriger und damit toxikologisch und dermatologisch unbedenklicher Konzentration in Kosmetikprodukten zum Einsatz gelangen können; Styrylresorcinol besitzt eine ca. um den Faktor 215 stärkere Tyrosinase-inhibierende Wirkung als Kojisäure.

Eigene Wirksamkeitsstudien mit synthetischem, gemäß literaturbekannten Verfahren hergestelltem Styrylresorcinol (Formel 4; CARN:85-27-8; 4-(1-Phenylethyl)-1,3-dihydroxybenzol) belegen beispielhaft, dass die Verbindungen der Formel 1 (Diphenylmethan-Derivate) sowie Substanzmischungen, die eine oder mehrere Verbindungen der Formel 1 umfassen, wobei die Gruppen R1 bis R5 jeweils die oben angegebene Bedeutung besitzen eine starke Tyrosinase-inhibierende Wirkung besitzen und somit hervorragend als hautaufhellende Mittel und als Mittel zur Bekämpfung von

Altersflecken einsetzbar sind. Aufgrund ihrer hohen Stabilität gegenüber Licht können sie dabei hervorragend als Alternative oder als Ergänzung zu bekannten hautaufhellenden Wirkstoffen (wie z.B Hydrochinon, Arbutin oder Ascorbinsäure) in Kosmetikprodukten und dergleichen als Hautaufheller Einsatz finden. Die Verbindung der Formel 4 und die weiteren Verbindungen der Formel 1, in denen die OH-Gruppen zueinander meta- oder para-ständig sind, sind darüber hinaus gegenüber Sauerstoff sehr stabil.

Die Tyrosinase-Inhibierung erfolgt regelmäßig aus kosmetischen Gründen, kann aber in Ausnahmefällen auch einen therapeutischen Charakter besitzen. Darüberhinaus können die Verbindungen der Formel 1 auch in der Lebensmittelindustrie oder in der Aromenindustrie als bräunungsinhibierende Zusätze zum Einsatz gelangen, siehe dazu unten.

Die Verbindungen der Formel 1 werden, insbesondere soweit sie als Mittel zur Haut- und Haarauhellung oder als Mittel zur Bekämpfung von Altersflecken eingesetzt werden, in der Regel topisch in Form von Lösungen, Cremes, Lotionen, Gelen, Sprays o.dgl. appliziert.

Wesentliche Anwendungsgebiete sind hierbei kosmetische, insbesondere dermatologische und/oder keratinologische Zubereitungen, die (abgesehen von der Anwesenheit von Verbindungen der Formel 1) wie üblich zusammengesetzt sind und dem kosmetischen, insbesondere dermatologischen und/oder keratinologischen Lichtschutz, zur Behandlung, der Pflege und der Reinigung der Haut und/oder der Haare oder als Schminkprodukt in der dekorativen Kosmetik dienen. Entsprechend können derartige Zubereitungen, je nach ihrem Aufbau beispielsweise als Hautschutzcreme, Reinigungsmilch, Reinigungsseife, Sonnenschutzlotion, Nährcreme, Tages- oder Nachtcreme, Deodorant, Antitranspirant, Shampoo, Haar-Pflegemiltel, Haar-Conditioner, Haar-Colorationen vewendet werden und dabei bevorzugt als Emulsion, Lotion, Milch, Creme, Hydrodispersionsgel, Balm, Spray, Schaum, Flüssigseife, Seifenstück, Haar-, Roll-on, Stick oder Make-up vorliegen.

Die erfindungsgemäßen Diphenylmethan-Derivate können darüberhinaus auch in Lebensmitteln eingesetzt werden. Besonders bevorzugte Produktkategorien sind hier insbesondere solche Nahrungsmittel, die aufgrund ihres natürlich vorkommenden Gehalts an phenolischen Verbindungen unter dem Einfluß von endogen vorhandenen Polyphenoloxidasen bei der Verarbeitung zu spontanen Bräunungsreaktionen neigen. Hierzu zählen vor allem Obst- und Gemüseprodukte, insbesondere Äpfel, Birnen oder Kartoffeln oder Krustentiere wie insbesondere Krabben, Langusten oder Shrimps, wobei diese Aufzählung natürlich nicht als vollständig zu erachten und beliebig erweiterbar ist.

Die Konzentration der Diphenylmethan-Derivate der Formel 1 in (insbesondere topisch zu applizierenden) Formulierungen liegt vorzugsweise im Bereich von 0,0001 bis 20 Gew.-%, bevorzugt im Bereich von 0,001 bis 5 Gew.-% und besonderes bevorzugt im Bereich von 0,01 bis 1 Gew.-%. Der Tyrosinase-inhibierende Wirkstoff kann hierbei (a) prophylaktisch oder (b) im Bedarfsfall zum Einsatz kommen.

Die Konzentration der z.B. täglich zu applizierenden Wirkstoffmenge ist unterschiedlich und hängt vom physiologischen Zustand des Probanden sowie individuatspezifischen Parametern wie Alter oder Körpergewicht ab. Diphenylmethan-Derivate der Formel 1 können allein, als Gemische oder auch in Kombination mit weiteren Tyrosinase-inhibierenden Substanzen zum Einsatz gelangen.

Die Verbindungen der Formel 1 (wobei R1 bis R5 die oben angegebenen Bedeutungen besitzen und auch hinsichtlich der bevorzugten Bedeutungen von R1 bis R5 das vorstehend Gesagte gilt) können auch als Bestandteil von kosmetischen Mitteln und Duftstoffkompositionen (Riechstoffkompositionen) eingesetzt werden und beispielsweise einem parfümierten (z.B. kosmetischen) Fertigprodukt eine Tyrosinase-inhibierende Wirkung verleihen. Eine besonders bevorzugte Duftstoffkomposition umfasst (a) eine sensorisch wirksame Menge eines Duftstoffes, (b) eine Tyrosinase-inhibierend wirkende Menge einer oder mehrerer Verbindungen der Formel 1 (wobei R1 bis R5 die oben angegebenen Bedeutungen besitzen können) sowie ggf. (c) einen oder mehrere Trägerstoffe und/oder Zusatzstoffe. Da der Anteil an Parfüm in einem kosmetischen Fertigprodukt häufig im Bereich von ca. 1 Gew.-% liegt, wird ein Parfüm, welches eine Verbindung der Formel 1 enthält, vorzugsweise zu etwa 5 bis 50 Gew.-% aus einer oder mehreren Verbindungen der Formel 1 bestehen. Als besonders vorteilhaft hatte sich erwiesen, dass die Substanzen der Formel 1 nur einen schwachen Eigengeruch besitzen oder gar völlig geruchlos sind; denn diese Eigenschaft prädestiniert sie für den Einsatz in einer Duftstoffkomposition.

Auch in einem bevorzugten Verfahren zur kosmetischen und/oder therapeutischen Hautaufhellung und zur Behandlung (Bekämpfung) von Altersflecken liegt die Einsatzkonzentration der erfindungsgemäßen, synergistisch wirksamen Mischungen im Bereich zwischen 0,0001 bis 20 Gew.-%, bevorzugt im Bereich zwischen 0,001 bis 5 Gew.-% und besonders bevorzugt im Bereich von 0,01 bis 1 Gew.-%, jeweils bezogen auf die Gesamtmasse des kosmetischen oder pharmazeutischen Produktes, welches die Mischung umfasst.

Die Diphenylmethan-Derivate der Formel 1 können hierbei (a) prophylaktisch oder (b) im Bedarfsfall zum Einsatz kommen.

Es sei darauf hingewiesen, dass der Begriff Diphenylmethan-Derivate im Rahmen des vorliegenden Textes bei den Derivaten der Formel 1, die unterschiedlich substituierte Phenylreste besitzen und für die gleichzeitig R2 und R3 unterschiedlich sind, auch die reinen S-konfigurierten Enantiomere, die R-konfigurierten Enantiomere sowie beliebige Mischungen aus S- und R-konfigurierten Enantiomeren umfasst. Aus kommerziellen Gründen ist es zwar in diesen Fällen besonders vorteilhaft, Gemische von Racematen der jeweiligen Diphenylmethan-Derivate zur Hautaufhellung

und/oder zur Bekämpfung von Altersflecken einzusetzen, da diese synthetisch besonders leicht zugänglich sind, die reinen Enantiomere oder nicht-racemische Mischungen dieser Enantiomere sind aber ebenfalls für die erfindungsgemäßen Zwecke geeignet.

Die erfindungsgemäß verwendeten Diphenylmethan-Derivate der Formel 1 lassen sich ohne Schwierigkeiten in gängige kosmetische oder dermatologische Formulierungen wie u.a. Pumpsprays, Aerosolsprays, Cremes, Salben, Tinkturen, Lotionen, Nagelpflegeprodukte (z.B. Nagellacke, Nagellackentferner, Nagelbalsame) und dergleichen einarbeiten. Hierbei ist es auch möglich und in manchen Fällen vorteilhaft, die erfindungsgemäß verwendeten Diphenylmethan-Derivate der Formel 1 mit weiteren Wirkstoffen zu kombinieren, beispielsweise mit anderen haut- und haaraufhellend wirkenden oder gegen Altersflecken wirkenden Stoffen. Die Diphenylmethan-Derivate der Formel 1 enthaltenden kosmetischen und/oder dermatologischen/keratologischen Formulierungen können hierbei ansonsten wie üblich zusammengesetzt sein und zur Behandlung der Haut und/oder der Haare im Sinne einer dermatologischen bzw. keratologischen Behandlung oder einer Behandlung im Sinne der pflegenden Kosmetik dienen. Sie können aber auch in Schminkprodukten in der dekorativen Kosmetik eingesetzt werden.

Kosmetische Zubereitungen, die Diphenylmethan-Derivate der Formel 1 enthalten, können auch weitere Wirkstoffe mit hautaufhellender Wirkung enthalten. Erfindungsgemäß können hierbei alle für kosmetische und/oder dermatologische Anwendungen geeigneten oder gebräuchlichen hautaufhellenden Wirkstoffe verwendet werden. Vorteilhafte hautaufhellende Wirkstoffe sind insoweit Kojisäure (5-Hydroxy-2-hydroxymetnyl-4-pyranon. Kojisäurederivate wie z.B. Kojisäuredipalmitat, Arbutin, Ascorbinsäure, Ascorbinsäurederivate, Hydrochinon, Hydrochinonderivate, Resorcin, schwefelhaltigen Moleküle wie z.B. Glutathion oder Cystein alpha-Hydroxysäuren. (z.B. Zitronensäure, Milchsäure, Apfelsäure) und deren Derivate, Cycloalkanone, Methylendioxyphenylalkanole, Vinyl- und Ethylgujacol, Inhibitoren der Stickstoffoxid-Synthese wie z.B., L-Nitroarginin und dessen Derivate, 2,7-Dinitroindazol oder Thiocitrullin, Metallchelatoren (z.B. $\alpha$-Hydroxyfettsäuren, Palmitinsäure, Phytinsäure, Lactoferrin, Huminsäure, Gallensäure, Gallenextrakte, Bilirubin, Biliverdin, EDTA, EGTA und deren Derivate), Flavonoide, Retinoide, Sojamilch, Serin-Protease-Inhibitoren oder Liponsäure oder andere synthetische oder natürliche Wirkstoffe zur Haut- und Haaraufhellung, wobei letztere auch in Form eines Extrakts aus Pflanzen, wie z.B. Bearberry-Extrakt, Reis-Extrakt, Süßholzwurzel-Extrakt oder daraus angereicherte Bestandteile wie Glabridin oder Licochalkon A, Artocarpus-Extrakt, Extrakt von Rumex- und Ramulus-Arten, Extrakte aus Kieferarten (Pinus) und Extrakte aus Vitis-Arten oder, daraus angereicherte Stilbenderivate, verwendet werden.

Die Diphenylmethan-Derivate der Formel 1 können in zahlreichen Fällen vorteilhaft in Kombination mit Konservierungsmittel eingesetzt werden. Vorzugsweise gewählt werden hierbei Konservierungsmittel wie Benzoesäure, ihre Ester und Salze, Propionsäure und ihre Salze, Salicylsäure und ihre Salze, 2,4-Hexadiensäure (Sorbinsäure) und ihre Satze, Formaldehyd und Paraformaldehyd, 2-Hydroxybiphenylether und seine Salze, 2-Zinksulfidopyridin-N-oxid, anorganische Sulfite und Bisulfite, Natriumjodat, Chlorbutanolum, 4-Ethylquecksilber-(II)5-Amino-1,3-bis(2-Hydroxybenzoesäure, ihre Salze und Ester, Dehydratcetsäure, Ameisensäure. 1,6-Bis(4-amidino-2-bromphenoxy)-n-hexan und seine Salze, das Natriumsalz der Ethylquecksilber-(II)-thioulicylsäure, Phenylquecksilber und seine Salze, 10-Undecylensäure und ihre Salze, 5-Amino-1,3-bis(2-ethylhexyl)-5-methyl-hexahydropyrimidin, 5-Brom-5-nitro-1,3-dioxan. 2-Brom-2-nitro-1.3-propandiol, 2,4-Dichlorbenrylalkohol, N-(4-Chlorphenyl)-N'-(3,4-dichlorphenyl)-hamstoff, 4-Chlor-m-kresol , 2,4,4'-Trichlor-2'-hydroxy-diphenylether, 4-Chlor-3,5-dimethylphenol. 1,1'-Methylen-bis(3-(1-hydroxymethyl-2,4-dioximidazolidin-5-yl)harnstoff), Poly-(hexamethylendiguanid)-hydrochlorid, 2-Phenoxyethanol, Hexamethylentetramin, 1-(3-Chloroallyl)-3,5,7-triaza-1-azonia-adamantanchlorid, 1(4-Chlorphenoxy)1(1H-imidazol-1-yl)-3,3-dimethyl-2-buta-non, 1.3-Bis-(hydroxy-methyl)-5,5-dimethyl-2,4-imidazolidindion, Benzylalkohol, Octopirox, 1,2-Dibrom-2,4-dicyanobutan, 2,2'-Methylen-bis(6-brom-4-chlor-phenol), Bromchlorophen, Mischung von 5-Chlor-2-methyl-3(2H)-isothiazolinon und 2-Methyl-3(2H)isothiazlinon mit Magnesiumchlorid und Magnesiumnitrat, 2-Benzyl-4-chlorphenol, 2-Chloracetamid, Chlorhexidin, Chlorhexidinacetat, Chlorhexidingluconat, Chlorhexidinhydrochlorid, 1-Phenoxy-propan-2-ol, N-Alkyl($C_{12}$-$C_{22}$)trimethylammoniumbromid und -chlorid, 4,4-Dimethyl-1,3-oxazo-lidin, N-Hydroxymethyl-N-(1,3-di(hydroxymethyl)-2,5-dioxoimidazolidin-4-yl)-N'-hydroxy-methylharnstoff, 1,6-Bis(4-amidino-phenoxy)-n-hexan und seine Salze, Glutaraldehyd, 5-Ethyl-1-aza-3,7-dioxabicyclo(3.3.0)octan, 3-(4-Chlorphenoxy)-1,2-propandiol, Hyamine, Alkyl-($C_8$-$C_{18}$)-dimethyl-benzyl-ammoniumchlorid, Alkyl-($C_8$-$C_{18}$)-dimethyl-benzylammonium-bromid, Alkyl-($C_8$-$C_{18}$)-dimethyl-benzylammoniumsaccharinat, Benzylhemiformal, 3-Jod-2-propinyl-butylcarbamat, Natrium-hydroxymethyl-aminoacetat oder Natrium-hydroxymethyl-aminoacetat.

In verschiedenen Fällen kann es auch vorteilhaft sein die Diphenylmethan-Derivate der Formel 1 in Kombination mit Substanzen einzusetzen, die vornehmlich zur Inhibition des Wachstums unerwünschter Mikroorganismen auf oder in tierischen Organismen eingesetzt werden. Erwähnenswert sind insoweit neben klassischen Konservierungsmitteln als weitere Wirkstoffe neben der großen Gruppe der klassischen Antibiotika insbesondere die für Kosmetika relevanten Produkte wie Triclosan, Climbazol, Octoxyglycerin, Octopirox (1-Hydroxy-4-methyl-6-(2,4,4-trimethylpentyl)-2(1H)-pyridone, 2-aminoethanol), Chitosan, Farnesol, Glycerinmonolaurat oder Kombinationen der genannten Substanzen, die u.a. gegen Achselgeruch, Fußgeruch oder Schuppenbildung eingesetzt werden.

Darüber hinaus können die Diphenylmethan-Derivate der Formel 1 auch in Kombination mit schweißhemmenden Wirkstoffen (Antitranspirantien) besonders vorteilhaft zur Bekämpfung von Körpergeruch eingesetzt werden. Als schweißhemmende Wirkstoffe kommen vor allem Aluminiumsalze wie Aluminiumchlorid, Aluminiumchlorhydrat, -nitrat, -sulfat, -acetat usw. zum Einsatz. Daneben kann aber auch die Verwendung von Zink-, Magnesium- und Zirkoniumverbindungen vorteilhaft sein. Für die Anwendung in kosmetischen und dermatologischen Antitranspirantien haben sich im wesentlichen die Aluminiumsalze und - in etwas geringerem Maße - Aluminium/Zirkoniumsalz-Kombinationen bewährt. Daneben erwähnenswert sind die teilneutralisierten und damit besser hautverträglichen, aber nicht ganz so wirksamen Aluminiumhydroxychloride. Neben Aluminiumsalzen kommen auch weitere Substanzen in Betracht wie zum Beispiel a) eiweißfällende Substanzen wie u.a. Formaldehyd, Glutaraldehyd, natürliche und synthetische Gerbstoffe sowie Trichloressigsäure, die einen oberflächlichen Verschluß der Schweißdrüsen herbeiführen b) Lokalanästhetika (u.a. verdünnte Lösungen von z.B. Lidokain, Prilokain oder Gemischen derartiger Substanzen), die durch Blockade der peripheren Nervenbahnen die sympathische Versorgung der Schweißdrüsen ausschalten, c) Zeolithe vom X-, A- oder Y-Typ, die neben der Reduktion der Schweißsekretion auch als Adsorbentien für schlechte Gerüche fungieren und d) Botulinustoxin (Toxin des Bakteriums *Chlostridium botulinum),* welches auch bei Hyperhidrose, einer krankhaft erhöhten Schweißsekretion, zum Einsatz gelangt und dessen Wirkung auf einer irreversiblen Blockierung der Freisetzung der für die Schweißsekretion relevanten Transmittersubstanz Acetylcholin beruht.

In manchen Fällen kann auch eine Kombination mit (Metall)-Chelatoren vorteilhaft sein. Bevorzugt einzusetzende (Metall)-Chelatoren sind hierbei u.a $\alpha$-Hydroxyfettsäuren. Phytinsäure, Lactoferrin, $\alpha$-Hyroxysäuren wie u.a. Zitronensäure, Milchsäure und Apfelsäure sowie Huminsäuren, Gallensäuren, Gallenextrakte, Bilirubin, Biliverdin bzw. EDTA, EGTA und deren Derivate.

Zur Anwendung werden die kosmetisch und/oder dermatologisch wirksamen Diphenylmethan-Derivate der Formel 1 in der für Kosmetika und Dermatika üblichen Weise auf die Haut und/oder die Haare in ausreichender Menge aufgebracht. Besondere Vorteile bieten dabei kosmetische und dermatologische Zubereitungen, die eine erfindungsgemäße Mischung enthalten und zusätzlich als Sonnenschutzmittel wirken. Vorteilhaft enthalten diese Zubereitungen mindestens einen UVA-Filter und/oder mindestens einen UVB-Filter und/oder mindestens ein anorganisches Pigment. Die Zubereitungen können dabei in verschiedenen Formen vorliegen, wie sie z.B. üblicherweise für Sonnenschutzzubereitungen eingesetzt werden. So können sie z.B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol darstellen.

Wie erwähnt können Zubereitungen, die Diphenylmethan-Derivate der Formel 1 enthalten, besonders vorteilhaft mit Substanzen kombiniert werden, die UV-Strahlung absorbieren, wobei die Gesamtmenge der Filtersubstanzen z.B. 0,01 Gew.-% bis 40 Gew.-%, vorzugsweise 0,1% bis 10 Gew.-%, insbesondere 1,0 bis 5,0 Gew.-% beträgt, bezogen auf das Gesamtgewicht der Zubereitungen, um kosmetische Zubereitungen zur Verfügung zu stellen, die das Haar bzw. die Haut vor ultravioletter Strahlung schützen. Vorteilhaft enthalten diese Zubereitungen mindestens einen UVA-Fitter und/oder mindestens einen UVB-Filter und/oder mindestens ein anorganisches Pigment, so dass ein Lichtschutzfaktor von zumindest > 2 (bevorzugt > 5) erreicht wird. Diese erfindungsgemäßen Zubereitungen können dabei in verschiedenen Formen vorliegen, wie sie z.B. üblicherweise für Sonnenschutzzubereitungen eingesetzt werden. So können sie z.B. eine Lösung, eine Emulsion vom Typ Wasser-in-Ol (W/O) oder vom Typ Öl-in-Wasser (O/W), oder eine multiple Emulsion, beispielsweise vom Typ Wasser-in-Ol-in-Wasser (W/O/W), ein Gel, eine Hydrodispersion, einen festen Stift oder auch ein Aerosol darstellen.

Enthalten die erfindungsgemäßen Zubereitungen UVB-Filtersubstanzen, können diese öllöslich oder wassertöslich sein. Vorteilhafte öllösliche UVB-Filter sind z.B.: 3-Benzylidencampher-Derivate, vorzugsweise 3-(4-Methylbenzyliden)campher, 3-Benzylidencampher; 4-Aminobenzoesäure-Derivate, vorzugsweise 4-(Dimethylamino)-benzoasäure(2-ethylhexyl)ester, 4-(Dimethylamino)benzoesäureamylester; Ester der Zimtsäure, vorzugsweise 4-Methoxyzimtsäure(2-ethylhexyl)ester, 4-Methoxyzimtsäureisopentylester, Ester der Salicylsäure, vorzugsweise Salicytsäure(2-ethylhexyl)ester, Salicylsäure(4isopropylbenzyl)ester, Salicylsäurehomomenthylester, Derivate des Benzo-phenons, vorzugsweise 2-Hydroxy-4-methoxybenzophenon, 2-Hydroxy-4-methoxy-4'-methylbenzophenon, 2,2'-Dihydroxy4-methoxybenzophenon; Ester der Benzalmalonsäure, vorzugsweise 4-Methoxybenzalmalonsäuredi(2-ethylhexyl) ester, 2,4,6-Trianilino-(p-carbo-2'-ethyl-1'-hexyloxy)1,3,5-triazin. Vorteilhafte wasserlösliche UVB-Filter sind z.B. Salze der 2-Phenylbenzimidazol-5-sulfonsäure wie ihr Natrium-, Kalium- oder ihr Triethanolammonium-Salz, sowie die Sulfonsäure selbst; Sulfonsäure-Derivate von Benzophenonen, vorzugsweise 2-Hydroxy-4-methoxybenzophenon-5-sulfansäure und ihre Salze; Sulfonsäure-Derivate des 3-Benzylidenoamphers, wie z.B. 4-(2-Oxo-3-bornylidenmethyl) benzolsulfonsäure, 2-Methyl-5-(2-oxo-3-bomylidenmethyl)-sulfonsäure und ihre Salze sowie das 1,4-di(2-oxo-10-Sulfo-3-bornylidenmethyl)-Benzol und dessen Salze (die entsprechenden 10-Sulfato-verbindungen, beispielsweise das entsprechende Natrium-, Kalium- oder Triethanolammonium-Salz), auch als Benzol-1,4-di(2-oxo-3-bomylidenmethyl-10-Sulfonsäure bezeichnet.

Die vorstehende Liste der genannten UVB-Filter, die in Kombination mit den Diphenylmethan-Derivaten der Formel 1 verwendet werden können, soll selbstverständlich nicht als abschließend verstanden werden. Es kann auch von Vorteil sein, UVA-Filter einzusetzen, wie sie üblicherweise in kosmetischen Zubereitungen enthalten sind. Bei diesen

Substanzen handelt es sich vorzugsweise um Derivate des Dibenzoylmethans, insbesondere um 1-(4'-tert-Butylphenyl)-3-(4'-methoxyphenyl) propan-1.3-dion und um 1-Phenyl-3-(4'-isopropylphenyl)propan-1,3-dion.

Die Diphenylmethan-Derivate der Formel 1 können in kosmetischen Zubereitungen vorteilhaft mit kosmetischen Hilfsstoffen kombiniert werden, wie sie üblicherweise in solchen Zubereitungen verwendet werden, also z.B. mit: Antioxidantien; Parfümölen; Mitteln zum Verhindern des Schäumens; Farbstoffen; Pigmente, die eine färbende Wirkung haben; Verdickungsmittel; oberflächenaktive Substanzen; Emulgatoren; weichmachende Substanzen; anfeuchtende und/oder feuchthaltende Substanzen; Fette; Ole; Wachse; andere übliche Bestandteile einer kosmetischen Formulierung wie Alkohole, Polyole, Polymere, Schaumstabilisatoren, Elektrolyte, organische Lösungsmittel oder Silikonderivate.

In Diphenylmethan-Derivate der Formel 1 enthaltenden Formulierungen zur topischen prophylaktischen oder kosmetischen Behandlung der Haut ist regelmäßig ein hoher Anteil an pflegenden Substanzen vorteilhaft. Gemäß einer bevorzugten Ausführungsform enthalten die Zusammensetzungen ein oder mehrere pflegende tierische und/oder pflanzliche Fette und Öle wie Olivenöl, Sonnenblumenöl, raffiniertes Sojaöl, Palmöl, Sesamöl, Rapsöl, Mandelöl, Borretschöl, Nachtkerzenöl, Kokosöl, Sheabutter, Jojobaöl, Spermöl, Rindertalg, Klauenöt und Schweineschmalz sowie gegebenenfalls weitere pflegende Bestandteile wie zum Beispiel Fettalkohole mit 8-30 C-Atomen. Die Fettalkohole können hierbei gesättigt oder ungesättigt bzw. linear oder verzweigt sein. Einsetzbar sind zum Beispiel Decanol, Decenol, Octanol, Octenal, Dodecanol. Dodecenol, Octadienol, Decadienol, Dodecadienol, Oleylalkohol, Ricinolalkohol, Erucaalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei die Aufzählung durch weitere strukturchemisch verwandte Alkohole nahezu beliebig erweiterbar wäre. Die Fettalkohole stammen bevorzugt von natürlichen Fettsäuren ab, wobei sie üblicherweise aus den korrespondierenden Estern der Fettsäuren durch Reduktion hergestellt werden. Einsetzbar sind weiterhin Fettalkoholfraktionen, die durch Reduktion aus natürlich vorkommenden Fetten und fetten Ölen, wie z.B. Rindertalg, Erdnußöl, Rüböl, Baumwollsamenöl, Sojaöl, Sonnenblumenöl, Palmkernöl, Leinöl, Maisöl, Rizinusöl, Rapsöl, Sesamöl. Kakaobutter und Kokosfett entstehen.

Zu pflegenden Substanzen, die sich vorzüglich mit den Diphenylmethan-Derivaten der Formel 1 kombinieren lassen, zählen darüber hinaus auch

- Ceramide, wobei man unter Ceramiden N-Acylsphingosine (Fettsäreamide des Sphingosins) oder synthetische Analoga solcher Lipide (sogenannte Pseudo-Ceramide) versteht, die das Wasserhaltevermögen des Stratum Comeums deutlich verbessern.
- Phospholipide, beispielsweise Sojalecithin, Ei-Lecithin und Kephaline
- Vaseline, Paraffin- und Silikonöle; zu letzteren zählen unter anderem Dialkyl- und Alkylarylsiloxane wie Dimethylpolysiloxan und Methylphenylpolysiloxan, sowie deren alkoxylierte und quaternierte Derivate.

Den Diphenylmethan-Derivaten der Formel 1 können vorteilhaft auch tierische und/oder pflanzliche Proteinhydrolysate zugesetzt werden. Vorteilhaft sind insoweit insbesondere Elastin-, Kollagen-, Keratin-, Milcheiweiß-, Sojaprotein-, Haferprotein-, Erbsenprotein-, Mandelprotein-, und Weizenproteinfraktionen oder entsprechende Proteinhydrolysate, aber auch deren Kondensationsprodukte mit Fettsäuren sowie quartemisierte Proteinhydrolysate, wobei die Verwendung pflanzlicher Proteinhydrolysate bevorzugt ist.

Sofern eine kosmetische oder dermatologische, Diphenylmethan-Derivate der Formel 1 enthaltende Zubereitung eine Lösung oder Lotion darstellt, können vorteilhafterweise als Lösungsmittel verwendet werden:

- Wasser oder wässrige Lösungen;
- fette Öle, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyt- oder -monobutylether, Diethylenglykolmonomethyl-oder -monoethylether und analoge Produkte. Insbesondere werden Gemische der vorstehend genannten Lösungsmittel verwendet. Bei alkoholischen Lösungsmitteln kann Wasser ein weiterer Bestandteil sein.

Kosmetische Zubereitungen, die Diphenylmethan-Derivate der Formel 1 enthalten, können auch Antioxidantien enthalten, wobei alle für kosmetische und/oder dermatologische Anwendungen geeigneten oder gebräuchlichen Antioxidantien verwendet werden können. Vorteilhaft werden die Antioxidantien gewählt aus der Gruppe bestehend aus

Aminosäuren (z.B. Glycin, Histidin. Tyrosin, Tryptophan) und deren Derivaten, Imidazolen (z.B. Urocaninsäure) und deren Derivaten, Peptiden wie D, L-Camosin, D-Camosin, L-Camosin und deren Derivaten (z.B. Anserin), Carotinoiden, Carotinen (z.B. α-Carotin, β-Carotin, Lycopin) und deren Derivaten, Uponsäure und deren Derivaten (z.B. Dihydroliponsäure), Aurothioglucose, Propylthiouracil und anderen Thiolen (z.B. Thioredoxin, Glutathion, Cystein, Cystin, Cystamin und deren Glycosyl-, N-Acetyl-. Methyl-, Ethyl-, Propyl-, Amyl-, Butyl- undLauryl-Lauryl-, Palmitoyl-, Oleyl-, γ-Linoleyl-, Cholesteryl- und Glycerylester) sowie deren Salzen, Dilaurylthiodipropionat, Distearylthiodipropionat. Thiodipropionsäure und deren Derivaten (Ester, Ether, Peptide, Lipide, Nukleotide, Nukleoside und Salze) sowie Sulfoximinverbindungen (z.B. Buthioninsutfoximine, Homocysteinsutfoximin, Buthioninsulfone, Penta-, Hexa-, Heptathioninsulfoximin) in sehr geringen verträglichen Dosierungen, ferner (Metall)-Chelatoren, z.B. α-Hydroxyfettsäuren, Palmitinsäure, Phytinsäure, Lactoferrin, α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und deren Derivate (z.B. Vitamin-Vitamin-E-acetat), Vitamin A und dessen Derivate (Vitarnin-A-palmitat) sowie Coniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO4) Selen und dessen Derivate (z.B. Selenmethionin), Stilbene und deren Derivate (z.B. Stilbenoxid, trans-Stilbenoxid) sowie Derivate (Salze, Ester, Ether, Zucker, Nukleotide, Nukleoside, Peptide und Lipide) dieser genannten Wirkstoffe.

Kosmetische Zubereitungen, die Diphenylmethan-Derivate der Formel 1 enthalten, können vorteilhafterweise auch Vitamine und Vitaminvorstufen enthalten, wobei alle für kosmetische und/oder dermatologische Anwendungen geeigneten oder gebräuchlichen Vitamine und Vitaminvorstufen verwendet werden können. Erwähnenswert sind hier insbesondere Vitamine und Vitaminvorstufen wie Tocopherole, Vitamin A, Niacinsäure und Niacinsäureamid, weitere Vitamine des B-Komplexes, insbesondere Biotin und Vitamin C, Panthenol und dessen Derivate, insbesondere die Ester und Ether des Panthenols sowie kationisch derivatisierte Panthenole wie z.B. Panthenoltriacetat, Panthenolmonoethytether und dessen Monoacetat sowie kationische Panthenolderivate.

Kosmetische Zubereitungen, die vorteilhafterweise Diphenylmethan-Derivate der Formel 1 enthalten, können auch entzündungshemmende und/oder rötungs- und/oder juckreizlindernde Wirkstoffe enthalten. Es können hierbei alle für kosmetische und/oder dermatologische Anwendungen geeigneten oder gebräuchlichen entzündungshemmenden bzw. rötungs- und/oder juckreizlindernden Wirkstoffe verwendet werden. Vorteilhaft werden als entzündungshemmende bzw. rötungs- und/oder juckreizlindernde Wirkstoffe steroidale entzündungshemmende Substanzen vom Kortikosteroiden-Typ eingesetzt wie z.B. Hydrocortison, Dexamethason, Dexamethasonphosphat, Methylprednisolon oder Cortison, wobei die Auflistung durch Zusatz weiterer steroidaler Entzündungshemmer erweiterbar ist. Auch nichtsteroidale Entzündungshemmer können eingesetzt werden. Beispielhaft erwähnt werden sollen hier Oxicame wie Piroxicam oder Tenoxicam; Salicylate wie Aspirin, Disalcid, Solprin oder Fendosal; Essigsäre-Derivate wie Diclofenac, Fenclofenac, Indomethacin, Sulindac, Tolmetin, oder Clindanac ; Fenamate wie Mefenamic, Meclofenamic, Flufenamic oder Niflumic ; Propionsäure-Derivate wie Ibuprofen, Naproxen, Benoxaprofen oder Pyrazole wie Phenylbutazon, Oxyphenylbutazon, Febrazon oder Azapropazon. Alternativ können natürliche entzündungshermmende bzw. rötungs- und/oder juckreizlindemde Stoffe eingesetzt werden. Einsetzbar sind Pflanzenextrakte, spezielle hochwirksame Pflanzenextrakt-Fraktionen sowie aus Pflanzenextrakten isolierte hochreine Wirksubstanzen. Besonders bevorzugt sind Extrakte, Fraktionen und Wirksubstanzen aus Kamille, Aloe vera, Commiphora-Arten, Rubia-Arten, Weiden, Weidenröschen, Hafer sowie Reinsubstanzen wie u.a. Bisabolol, Apigenin-7-glucosid, Boswelliasäure, Phytosterole, Glycyrrhizinsäure, Glabridin oder Licochalkon A. Die Diphenylmethan-Derivate der Formel 1 enthaltenden Formulierungen können auch Gemische aus zwei oder mehreren antiinflammatorischen Wirkstoffen enthalten.

Besonders bevorzugt zur Verwendung im Sinne der Erfindung sind Bisabolol, Boswelliasäure, sowie Extrakte und isolierte hochreine Wirkstoffe aus Hafer und Echinacea, insbesondere bevorzugt sind α-Bisabolol und Extrakte und isolierte hochreine Wirkstoffe aus Hafer.

Die Menge der Antiimitantien (eine oder mehrere Verbindungen) in den Zubereitungen beträgt vorzugsweise 0,0001 bis 20 Gew.-%, besonders bevorzugt 0,0001 - 10 Gew.-%, insbesondere 0,001 - 5 Gew.-%, bezogen auf das Gesamtgewicht der Zubereitung.

Kosmetische Zubereitungen, die Diphenylmethan-Derivate der Formel 1 enthalten, können vorteilhafterweise auch Feuchthalteregulatoren enthalten. Als Feuchthalteregulatoren ("moisturizer") finden z.B. folgende Stoffe Verwendung: Natriumlactat, Harnstoff, Alkohole, Sorbit, Glycerin, Propylenglykol, Kollagen, Elastin oder Hyaluronsäure, Diacyladipate, Petrolatum, Ectoin, Urocaninsäure, Lecithin, Pantheol, Phytantriol, Lycopen, Algen-Extrakt, Ceramide, Cholesterol, Glykolipide, Chitosan, Chondroitinsulfat, Polyaminosäuren und -zucker, Lanolin, Lanolinester, Aminosäuren, alpha-Hydroxysäuren (z.B. Zitronensäure, Milchsäure, Apfelsäure) und deren Derivate, Zucker (z.B. Inositol), alpha-Hydroxy-Fettsäuren, Phytosterole, Triterpensäuren wie Betulinsäure oder Ursolsäure, Algenextrakte.

Kosmetische Zubereitungen, die Diphenylmethan-Derivate der Formel 1 enthalten, können vorteilhafterweise

auch Mono-, Di- und Oligosaccharide wie beispielsweise Glucose, Galactose, Fructose, Mannose, Fruchtzucker und Lactose enthalten.

Kosmetische Zubereitungen, die Diphenylmethan-Derivate der Formel 1 enthalten, können vorteilhafterweise auch Pflanzenextrakte enthalten, die üblicherweise durch Extraktion der gesamten Pflanze, in einzelnen Fällen aber auch ausschließlich aus Blüten und/oder Blättern, Holz, Rinde oder Wurzeln der Pflanze hergestellt werden. Hinsichtlich der verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflegemittel und Waschmittel e.V. (IKW), Frankfurt beginnenden Tabelle aufgeführt sind. Vorteilhaft sind insbesondere die Extrakte aus Aloe, Hamamelis, Algen, Eichenrinde, Weidenröschen, Brennnessel, Taubnessel, Hopfen, Kamille, Schafgarbe, Arnika, Calendula, Klettenwurzel, Schachtelhalm, Weißdorn, Lindenblüten, Mandel, Fichtennadel, Rosskastanie, Sandelholz, Wacholder, Kokosnuss, Mango, Aprikose, Orange, Zitrone, Limette, Grapefruit, Apfel, grünem Tee, Grapefruitsamen, Weizen, Hafer, Gerste, Salbei, Thymian, Quendel, Rosmarin, Birke, Malve, Wiesenschaumkraut, Weidenrinde, Hauhechel, Huflattich, Eibisch, Ginseng und Ingwerwurzel. Besonders bevorzugt sind dabei die Extrakte aus Aloe vera, Kamille, Algen, Rosmarin, Calendula, Ginseng, Gurke, Salbei, Brennnessel, Lindenblüten, Amika, und Hamamelis. Es können auch Mischungen aus zwei oder mehreren Pflanzenextrakten eingesetzt werden. Als Extraktionsmittel zur Herstellung der genannten Pflanzenextrakte können u.a. Wasser, Alkohole sowie deren Mischungen verwendet werden. Unter den Alkoholen sind dabei niedere Alkohole wie Ethanol und Isopropanol, aber auch mehrwertige Alkohole wie Ethylenglykol, Propylenglykol und Butylenglykol bevorzugt, und zwar sowohl als alleiniges Extraktionsmitttel als auch in Mischungen mit Wasser. Die Pflanzenextrakte können sowohl in reiner als auch in verdünnter Form eingesetzt werden.

Kosmetische Zubereitungen, die Diphenylmethan-Derivate der Formel 1 enthalten, können, insbesondere wenn kristalline oder mikrokristalline Festkörper, beispielsweise anorganische Mikropigmente in die Zubereitungen eingearbeitet werden sollen, auch anionische, kationische, nichtionische und/oder amphotere Tenside enthalten. Tenside sind amphiphile Stoffe, die organische, unpolare Substanzen in Wasser lösen können. Bei den hydrophilen Anteilen eines Tensidmoleküls handelt es sich dabei meist um polare funktionelle Gruppen, beispielweise -COO, $-OSO_3^{2-}$, $-SO_3^-$, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:

- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch. Typisch für nicht-ionische Tenside sind Polyether-Ketten. Nicht-ionische Tenside bilden in wässrigem Medium keine Ionen.

A. Anionische Tenside

Vorteilhaft zu verwendende anionische Tenside sind Acylaminosäuren (und deren Salze), wie

- Acylglutamate, beispielsweise Natriumacylglutamat, Di -TEA-palmitoylaspartat und Natrium Capryl/ Capringlutamat,
- Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
- Sarcosinate, beispielsweise Myristoyl Sarcosin, TEA-lauroylsarcosinat, Natriumlauroylsarcosinat und Natriumcocoylsarkosinat,
- Taurate, beispielsweise Natriumlauroyltaurat und Natriummethylcocoyltaurat,

- Acyllactylate, Lauroyllactylat, Caproyllactylat
- Alaninate

Carbonsäuren und Derivate, wie

- beispielsweise Laurinsäure, Aluminiumstearat, Magnesiumalkanolat und Zinkundecylenat,
- Ester-Carbonsäuren, beispielsweise Calciumstearoyllactylat, Laureth-6 Citrat und Natrium PEG-4 Lauramidcarboxylat,
- Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,
**[0059]** Sulfonsäuren und Salze, wie

- Acyl-isothionate, z.B. Natrium-/Ammoniumococoyl-isethionat,
- Alkylarylsulfonate,
- Alkylsulfonate, beispielsweise Natriumcocosmonoglyceridsulfat, Natrium $C_{12-14}$ Olefin-sulfonat, Natriumlaurylsulfoacetat und Magnesium PEG-3 Cocamidsulfat,
- Sulfosuccinate, beispielsweise Dioctylnabriumsulfosuccinat, Dinatriumlaureth-sulfosuocinat, Dinatriumlaurylsulfosuccinal und Dinatriumundecylenamido MEA-Sulfosuccinat

sowie
Schwefelsäureester, wie

- Alkylethersulfat, beispielsweise Natrium-, Ammonium-, Magnesium-, MIPA, TIPA-Laurethsulfat, Natriummyrethsulfat und Natrium C12-13 Parethsulfat,
- Alkylsulfate, beispielsweise Natrium-, Ammonium- und TEA-Laurylsulfat.

B. Kationische Tenside

Vorteilhaft zu verwendende kationische Tenside sind

- Alkylamine,
- Alkylimidazole,
- Ethoxylierte Amine und
- Quaternäre Tenside.
  $RNH_2CH_2CH_2COO$ (bei pH=7)
  $RNHCH_2CH_2COO^-$ $B^+$ (bei pH=12) $B^+$ = beliebiges Kation, z.B. $Na^+$
- Esterquats
  Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkylamidopropylhydroxysulfain. Die verwendeten kationischen Tenside können ferner bevorzugt gewählt werden aus der Gruppe der quaternären Ammoniumverbindungen, insbesondere Benzyltrialkyl-ammoniumchloride oder -bromide, wie beispielsweise Benzyldimethylstearyl-ammoniumchlorid, ferner Alkyltrialkylarnrmniumsatze, beispielsweise Cetylirimethylammoniumchlorid oder -bromid, Alkyldimethylhydroxy-ethylammoniumchloride oder -bromide, Dialkyldimethylammoniumchloride oder -bromide, Alkylamidethyltrimethyl-ammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide.

Vorteilhaft sind insbesondere Cetyltrimethyl-ammoniumsalze zu verwenden.

C. Amphotere Tenside

Vorteilhaft zu verwendende amphotere Tenside sind

- Acyl-/dialkylethylendiamin, beispielsweise Natriumacylamphoacetat, Dinatriumacylamphodipropionat, Dinatriumalkylamphodiacetat, Natriumacytamphohydroxy-propylsulfonat, Dinatriumacylamphodlacetat und Natriumacylamphopropionat,

- N-Alkylaminosäuren, beispielsweise Aminopropylalkylglutamid, Alkylaminopropionsaure, Natriumalkylimidodipropionat und Lauroamphocarboxyglycinat.

D. Nicht-ionische Tenside

Vorteilhaft zu verwendende nicht-ionische Tenside sind

- Alkohole,

- Alkanolamide, wie Cocamide MEA/ DEA/ MIPA,

- Aminoxide, wie Cocoamidopropylaminoxid,

- Ester, die durch Veresterung von Carbonsäuren mit Ethylenoxid, Glycerin, Sorbitan oder anderen Alkoholen entstehen,

- Ether, beispielsweise ethoxylierte/propoxylierte Alkohole, ethoxylierte/propoxylierte Ester, ethoxylierte/ propoxylierte Glycerinester, ethoxylierte/ propoxy-lierte Cholesterine, ethoxylierte/ propoxylierte Triglyceridester, ethoxyliertes prop-oxyliertes Lanolin, ethoxylierte/propoxylierte Polysiloxane, propoxylierte POE-Ether und Alkylpolyglycoside wie Laurylglucosid, Decylglycosid undCocoglyco-sid.

- Sucroseester, -Ether

- Polyglycerinester, Diglycerinester, Monoglycerinester

- Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Die oberflächenaktive Substanz kann in einer Konzentration zwischen 1 und 98 Gew.-% in den erfindungsgemäßen, Diphenylmethan-Derivate der Formel 1 enthaltenden Zubereitungen vorliegen, bezogen auf das Gesamtgewicht der Zubereitungen.

Kosmetische oder dermatologische Zubereitungen, die erfindungsgemäße Diphenylmethan-Derivate der Formel 1 enthalten, können auch als Emulsionen vorliegen.

Die Ölphase kann vorteilhaft gewählt werden aus folgender Substanzgruppe:

- Mineralöle, Mineralwachse

- fette Öle, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;

- Alkylbenzoate;

- Silikonöle wie Dimethylpolysiloxane, Diethylpolysiloxane, Diphenylpolysiloxane sowie Mischformen daraus.

Vorteilhaft einsetzbar sind (a) Ester aus gesättigten und/oder ungesättigten verzweigten und/oder unverzweigten Alkancarbonsäuren einer Kettenlänge von 3 bis 30 C-Atomen und gesättigten und/oder ungesättigten, verzweigten

und/oder unverzweigten Alkoholen einer Kettenlänge von 3 bis 30 C-Atomen, (b) Ester aus aromatischen Carbonsäuren und gesättigten und/oder ungesättigten, verzweigten und/oder unverzweigten Alkoholen einer Kettenlänge von 3 bis 30 C-Atomen. Bevorzugte Esteröle sind Isopropylmyristat, Isopropylpalmitat. Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethythexyllaurat, 2-Hexytdecytrtearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr. Auch beliebige Abmischungen solcher Öl- und Wachskomponenten sind vorteilhaft einsetzbar. In manchen Fällen ist es auch vorteilhaft, Wachse, beispielsweise Cetylpalmitat, als alleinige Lipidkomponente der Ölphase einzusetzen, vorteilhaft wird die Ölphase gewählt aus der Gruppe , die besteht aus 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid und Dicaprylylether. Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat. Auch die Kohlenwasserstoffe Paraffinöl, Squalan und Squalen lassen sich vorteilhaft verwenden. Vorteilhaft kann die Ölphase ferner einen Gehalt an cydischen oder linearen Silikonölen aufweisen oder vollständig aus solchen Ölen bestehen, wobei es allerdings bevorzugt ist, außer dem Silikonöl oder den Silikonölen einen zusätzlichen Gehalt an anderen Ölphasenkomponenten zu verwenden. Cyclomethicon (z.B. Decamethylcyclopentasiloxan) kann vorteilhaft als Silikonöl eingesetzt werden. Aber auch andere Silikonöle sind vorteilhaft verwendbar, beispielsweise Undecamethylcydotrisiloxan, Polydimethylsiloxan und Poly(methylphenylsiloxan). Besonders vorteilhaft sind ferner Mischungen aus Cyclomethicon und Isotridecylisononanoat sowie aus Cyclomethicon und 2-Ethylhexylisostearat.

Die wässrige Phase von als Emulsion vorliegenden Zubereitungen, die Diphenylmethan-Derivate der Formel 1 enthalten, kann vorteilhafterweise umfassen: Alkohole, Diole oder Polyole niedriger C-Zahl, sowie deren Ether, vorzugsweise Ethanol, Isopropanol, Propylenglykol, Glycerin, Ethylenglykol, Ethylenglykol- monoethyl- oder -monobutylether, Propylenglykolmonomethyl, - monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropyl-methylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Als Emulsion vorliegende Zubereitungen, die erfindungsgemäße Diphenylmethan-Derivate der Formel 1 enthalten, umfassen vorteilhaft einen oder mehrere Emulgatoren. O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:

- der Fettalkoholethoxylate
- der ethoxylierten Wollwachsalkohole,
- der Polyethylenglycolether der allgemeinen Formel $R-O-(-CH_2-CH_2O-)_n-R'$,
- der Fettsäureethoxylate der allgemeinen Formel $R-COO-(-CH_2-CH_2-O-)_n-H$,
- der veretherten Fettsäureethoxylate der allgemeinen Formel

  $$R-COO-(-CH_2-CH_2-O-)_n-R',$$

- der veresterten Fettsäureethoxylate der allgemeinen Formel

  $$R-COO-(-CH_2-CH_2-O-)_n-C(O)-R',$$

- der Polyethylenglycolglycerinfettsäureester

- der ethoxylierten Sorbitanester
- der Cholesterinethoxylate
- der ethoxylierten Triglyceride
- der Alkylethercarbonsäuren der allgemeinen Formel

$$R\text{-}COO\text{-}(\text{-}CH_2\text{-}CH_2\text{-}O\text{-})_n\text{-}OOH,$$

und n eine Zahl von 5 bis 30 darstellen,

- der Polyoxyethylensorbitolfettsäureester,
- der Alkylethersulfate der allgemeinen Formel $R\text{-}O\text{-}(\text{-}CH_2CH_2\text{-}O\text{-})_n\text{-}SO_3\text{-}H$
- der Fettalkoholpropoxylate der allgemeinen Formel $R\text{-}O\text{-}(\text{-}CH_2CH(CH_3)\text{-}O\text{-})_n\text{-}H$
- der Polypropylenglycolether der allgemeinen Formel

$$R\text{-}O\text{-}(\text{-}CH_2\text{-}CH(CH_3)\text{-}O\text{-})_n\text{-}R'$$

- der propoxylierten Wollwachsalkohole,
- der veretherten Fettsäurepropoxylate $R\text{-}COO\text{-}(\text{-}CH_2\text{-}CH(CH_3)\text{-}O\text{-})_n\text{-}R'$
- der veresterten Fettsäurepropoxylate der allgemeinen Formel

$$R\text{-}COO\text{-}(\text{-}CH_2\text{-}CH(CH_3)\text{-}O\text{-})_n\text{-}C(O)\text{-}R$$

- der Fettsäurepropoxylate der allgemeinen Formel

$$R\text{-}COO\text{-}(\text{-}CH_2\text{-}CH(CH_3)\text{-}O\text{-})_n\text{-}H,$$

- der Polypropylenglycolglycerinfettsäureester
- der propoxylierten Sorbitanester
- der Cholesl erinpropoxylate
- der propoxylierten Triglyceride
- der Alkylethercarbonsäuren der allgemeinen Formel

$$R\text{-}O\text{-}(\text{-}CH_2\text{-}CH(CH_3)\text{-}O\text{-})_n\text{-}CH_2\text{-}COOH,$$

- der Alkylethersulfate bzw- die diesen Sulfaten zugrundeliegenden Säuren der allgemeinen Formel $R\text{-}O\text{-}(\text{-}CH_2CH(CH_3)\text{-}O\text{-})_n\text{-}SO_3\text{-}H$,
- der Fettalkoholethoxylate/propoxylate der allgemeinen Formel $R\text{-}O\text{-}X_n\text{-}Y_m\text{-}H$
- der Polypropylenglycolether der allgemeinen Formel $R\text{-}O\text{-}X_n\text{-}Y_m\text{-}R'$
- der veretherten Fettsäurepropoxylate der allgemeinen Formel $R\text{-}COO\text{-}X_n\text{-}Y_m\text{-}R'$
- der Fettsäureethoxylate/propoxylate der allgemeinen Formel $R\text{-}COO\text{-}X_n\text{-}Y_m\text{-}H$.

Erfindungsgemäß besonders vorteilhaft werden die eingesetzten polyethoxylierten bzw. polypropoxylierten

bzw. polyethoxylierten und polypropoxylierten O/W-Emulgatoren gewählt aus der Gruppe der Substanzen mit HLB-Werten von 11 - 18, ganz besonders vorteilhaft mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol (15)stearylether (Steateth-15), Polyethylenglycol(16)stearylether (Steareth-16), Polyethylenglycol(17)stearylether (Steareth-17), Polyethylenglycol-(18)stearylether (Steareth-18), Polyethylenglycol(19)stearylether (Steareth-19), Polyethylenglycol(20)stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol (17)isostearylether (lsosteareth-17), Polyethylenglycol-(18)isostearylether (Isosteareth-18), Polyethylenglycol(19) isostearylether (Isosteareth-19), Polyethylenglycol(20)isostearylether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)catylether (Ceteth-16), Polyethylenglycol(17)cetylether (Ceteth-17), Polyethylenglycol(18)cetylether (ceteth-18), Polyethylenglycol(19)cetylether (Ceteth-19), Polyethylenglycol-(20)cetylether (Ceteth-20), Polyethylenglycol(13)isocetylether (Isoceteth-13), Polyethylenglycoi(14)isocatylether (Isoceteth-14), Polyethylenglycol (15)isocetylether (lsoceteth-15), Polyethylenglycol(16)isocetylether (Isocatoth-16), Polyethylenglycol(17)isocetylether (Iscoototh-17), Polyethylenglycol(18)isocetylether (isomteth-18), Polyethylenglycol(19)isocetylether(Isoceteth-19). Polyethylenglycol(20)isocetylether (lsoceteth-20), Polyathylenglyool(12)oleylether (Oleth-12), Polyethylenglycol(13)oleylether (Oleth-13), Polyethylenglycol(14)-oleylether (Oleth-14), Polyethylenglycol(15)oleylether (Oleth-15), Polyethylenglycol(12)laurylether (Laureth-12), Polyethylenglycol(12)isolaurylether (isolaureth12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol (17)cetylstearylether (Ceteareth-17), Polyethylenglycol-(18)cetylstearylether (Ceteareth-18), Polyethylenglycol-(19) cetylstearylether (Ceteareth-19), Polyethylenglycol(20)cetylstearylether (Ceteareth-20),

Es ist ferner von Vorteil, die Fettsäureethoxylate aus folgender Gruppe zu wählen:

Polyethylenglycol(20)stearat, Polyethylenglycol(21)stearat, Polyethylengtycol-(22)stearat, Polyethylenglycol(23) stearat, Polyethylenglycol(24)stearat, Polyethylenglycol(25)stearat, Polyethylenglycol(12)isostearat, Polyethylenglycol-(13)isostearat, Polyethylenglycol-(14)isostearat, Polyethylenglycol(15)isostearat, Polyethylenglycol(16)isostearat, Polyemylenglycol(17)isostearet, Polyethylenglycol(18)isostearat, Polyethylenglycol(19)isostearat, Polyethylenglycol(20)isostearat, Polyothylenglycol(21)isostearat Polyethylenglycol-(22)isostearat, Polyethylenglycol(23) isostearat, Polyethylenglycol(24)isostearat, Polyethylenglycol(25)isostearat. Polyethylenglycol(12)oleat, Polyethylenglycol(13)-oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Satz kann vorteilhaft das Natriumlaureth-11-carboxylat verwendet werden. Als Alkylethersulfat kann Natrium Laureth 1-4 sulfat vorteilhaft verwendet werden. Als ethoxyliertes Cholesterinderivat kann vorteilhaft Polyethylenglycol(30)Cholesterylether verwendet werden. Auch Polyethylenglycol (25)Sojasteral hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20) glyceryllaurat. Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanrnonoisostearat, Polyethylenglycol(20)sorbitanmonopalmitat, Polyethyleriglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 bis 18 C-Atomen, Diglycerinester gesättigter und/oder ungesättigter, verzweigter und/oder unverzweigter Alkancarbonsäuren einer Kettenlänge von 8 bis 24, insbesondere 12 bis 18 C-Atomen, Monoglycerinether gesättigter und/oder ungesättigter, verzweigter und/oder unverzweigter Alkohole einer Kettenlänge

von 8 bis 24, insbesondere 12 bis 18 C-Atomen, Diglycerinether gesättigter und/oder ungesättigter, verzweigter und/oder unverzweigter Alkohole einer Kettenlänge von 8 bis 24, insbesondere 12 bis 18 C-Atomen, Propylenglycol-ester gesättigter und/oder ungesättigter, verzweigter und/oder unverzweigter Alkancarbonsäuren einer Kettenlänge von 8 bis 24, insbesondere 12 bis 18 C-Atomen sowie Sorbitanester gesättigter und/oder ungesättigter, verzweigter und/oder unverzweigter Alkancarbonsäuren einer Kettenlänge von 8 bis 24, insbesondere 12 bis 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglyoolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Bevorzugte Ausgestaltungen und weitere Aspekte der vorliegenden Erfindung ergeben sich aus den beigefügten Patentansprüchen und den nachfolgenden Beispielen.

Beispiel 1:

Untersuchungen zur Tyrosinase-inhibierenden Wirkung von Styrylresorcinol (4)

Die Erkenntnis, dass Diphenylmethan-Derivate der Formel 1 (wobei R 1-5 jede der oben angegebenen Bedeutungen besitzen kann) hervorragend als Mittel zur Hautaufhellung und zur Bekämpfung von Altersflecken verwendet werden können geht aus den folgenden Untersuchungen hervor, die an 3T3 Fibrosarcoma-Zellen oder B16V Maus Melanoma-Zellen durchgeführt wurden

A. (Zytotoxizitäts-Bestimmung)

3T3 Fibrosarcoma-Zellen oder B16V Maus Melanoma-Zellen werden in eine 96well-Mikrotitierplatte in einer Konzentration von 1 x $10^4$ Zellen/Well (3T3) bzw. 2 x $10^4$ Zellen/Well (B16V) ausgesät. Nach 24 h Kultivierung bei 37°C und 5 % $CO_2$ in DMEM-Medium (3T3-Zellen) bzw. RPMI-Medium (B16V-Zellen), angereichert mit 10 % fötalem Kälberserum, wird das Medium abgezogen. Es werden verschiedene Konzentrationen der Testsubstanzen, gelöst in frischem Medium angereichert mit 5 % fötalem Kälberserum, zugegeben und für weitere 48 h inkubiert. Parallel wird mit SDS als Standard in Konzentrationen von 0,01 mM, 0,1 mM, 1 mM und 10 mM inkubiert. Nach der Inkubation wird das Medium abgezogen und die Zellen werden für mit 2 h mit MTT (3-[4,5-Dimethylthiazol-2-yl]2,5-diphenyl Tetrazolium Bromid) inkubiert. Nach Extraktion des Farbstoffs mit essigsaurem SDS in DMSO (10 min) wird die Absorption (A) bei 570 nm gemessen.

Es werden Mittelwert und Standardabweichung der Kontrollen, der Blanks und der Proben berechnet Der Mittelwert des Blanks wird von den Mittelwerten der Kontrollen und Proben subtrahiert. Die Viabilität der Zellen wird prozentual in Bezug auf die Kontrollen (100 %) angegeben:

$$\text{Viabilität (\%)} = (A_{Testverbindung}/A_{Kontrolle}) \times 100]$$

Tabelle 1: Zytotoxizität (MTTILDH-Assay, 3T3 Fibroblasten und B16V Maus Melanomazellen) - $IC_{50}$-und $IC_{20}$-Werte für Styrylresorcinol, 4-Hexylresorcinol und Kojisäure.

| | 3T3-Zellen | | B16V-Zellen | |
|---|---|---|---|---|
| **Testsubstanz** | **$IC_{20}$** | **$IC_{50}$** | **$IC_{20}$** | **$IC_{50}$** |
| Kojisäure | > 100 mM | > 100 mM | > 100 mM | > 100 mM |
| 4-Hexytresorcinol | 0,07 mM | 0,14 mM | n.b. | n.b. |
| Styrylresorcinol (4) | 0,07 mM | 0,15 mM | 0,02 mM | 0,14 mM |
| n.b.: nicht bestimmt | | | | |

Die cytotoxikologischen Untersuchungen zeigen, dass Styrylresorcinol eine verhältnismäßig geringe Cytotoxizität besitzt, die in etwa vergleichbar ist mit der Cytotoxizität von 4-Hexylresorcinol, welches u.a. auch in der Lebensmittelchemie zum Einsatz gelangt.

B: $IC_{50}$-Werte für Styrylresorcinol (4) im Vergleich zu Kojisäure und 4-Hexylresorcinol

Die $IC_{50}$-Werte von Styrylresorcinol (4), Kojisäure und 4-Hexylresorcinol wurden gemäß den nachfolgend beschriebenen allgemeinen Testbedingungen bestimmt und sind in Tabelle 2 zusammengefasst.

Versuchsdurchführung: B16V Maus Melanoma-Zellen werden in eine 96well-Mikrotitierplatte in einer Konzentration von $5 \times 10^3$ Zellen/well ausgesät. Nach 24 h Kultivierung bei 37°C und 5 % $CO_2$ in RPMI-Medium, angereichert mit 10 % fötalem Kälberserum, werden verschiedene Konzentrationen der Testsubstanzen sowie 10 nM α-MSH (α-Melanocyte Stimulating Hormone) zugegeben und für weitere 96 h inkubiert. Die maximale Einsatzkonzentration der Testsubstanzen entspricht dem 0,1 fachen Wert des jeweiligen $IC_{20}$-Wertes des Zytotoxizitäts-Assays. Parallel wird mit Kojisäure als Standard in Konzentrationen von 0,01 mM, 0,1 mM und 1 mM inkubiert. Nach der Inkubation wird das Kulturmedium mit SDS und NaOH (Endkonzentrationen: 1mM bzw. 1M) versetzt und die Absorption (A) nach 3 h bei 400 nm gemessen.

Die Inhibition der Pigmentierung in Anwesenheit der Testverbindungen oder der Kojisäure wurden gemäß der folgenden Gleichung berechnet:

$$\text{Inhibition der Pigmentierung (\%)} = 100 - [(A_{Testverbindung}/A_{Kontrolle}) \times 100]$$

Aus der Inhibition der Pigmentierung (%) in einer Reihe von Verdünnungen von Testverbindungen wird für jede Testverbindung der $IC_{50}$ berechnet. Dies ist die Konzentration einer Testverbindung, bei der die Pigmentierung zu 50 % gehemmt wird.

Tabelle 2: Lightening-Wirkung (B16V Maus Melanomazellen)-$IC_{50}$-Werte für Styrylresorcinol, 4-Hexylresorcinol und Kojisäure

| **Testsubstanz** | **$IC_{50}$ (μM)** |
|---|---|
| Kojisäure | 452,3 |
| Styrylresorcinol (4) | 2,1 |
| 4-Hexylresorcinol | 5,2 |

Wie Tabelle 2 zeigt, hemmt Styrylresorcinol in sehr niedrigen Konzentrationen signifikant die Tyrosinase-Aktivität und kann somit hervorragend als Mittel zur Haut-und Haaraufhellung und zur Bekämpfung von Altersflecken zum Einsatz gelangen. Im Vergleich zu Kojisäure, einem in kosmetischen Produkten bereits häufig verwendeten Wirkstoff zur Haut-und Haaraufhellung bzw. zur Bekämpfung von Altersflecken, welcher jedoch toxikologisch nicht ganz unbedenklichen ist, zeigt das erfindungsgemäße Styrylresorcinol (Formel 4) eine um einen Faktor von ca. 215 stärkere Wirksamkeit Gegenüber 4-Hexylresorcinol erweist sich das Styrylresorcinol (Formel 4) immer noch mehr als doppelt so stark wirksam. Die Wirkung des 4-Hexylresorcinols gegenüber Kojisäure ist demzufolge auch nur etwa 87mal so stark.

Die vorstehend diskutierten Untersuchungen zeigen eindeutig, dass Diphenylmethan-Derivate der Formel 1 (wobei R1 bis R5 die oben angegebenen Bedeutungen besitzen und auch hinsichtlich der bevorzugten Bedeutungen von R1 bis R5 das vorstehend Gesagte gilt) Tyrosinase stark inhibieren und somit hervorragend als haut- und haaraufhellende Mittel, zur Bekämpfung von Altersflecken und/oder als Bräunungsinhibitoren in der Lebensmittelchemie eingesetzt werden können.

## Patentansprüche

1. Verwendung einer Verbindung der Formel 1 oder einer Substanzmischung, die eine oder mehrere Verbindungen der Formel 1 umfasst,

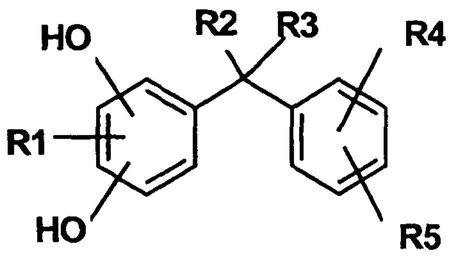

**1**

wobei gilt:

R1 ist

- Wasserstoff,
- Methyl,
- geradkettiges oder verzweigtes, gesättigtes oder ungesättigtes Alkyl mit 2-4 C-Atomen
- OH oder
- Halogen,

R2 ist

- Wasserstoff,
- Methyl oder
- geradkettiges oder verzweigtes, gesättigtes oder ungesättigtes Alkyl mit 2-5 C-Atomen,

R3 ist

- Methyl oder
- geradkettiges oder verzweigtes, gesättigtes oder ungesättigtes Alkyl mit 2-5 C-Atomen,

und
R4 und R5 sind unabhängig voneinander

- Wasserstoff,
- Methyl,
- geradkettiges oder verzweigtes, gesättigtes oder ungesättigtes Alkyl mit 2-5 C-Atomen
- OH oder
- Halogen

als Tyrosinase-Inhibitor zur Haaraufhellung und/oder als Bräunungsinhibitor in der Lebensmittelindustrie.

**2.** Verwendung einer Verbindung der Formel 1 oder einer Substanzmischung, die eine oder mehrere Verbindungen der Formel 1 umfasst,

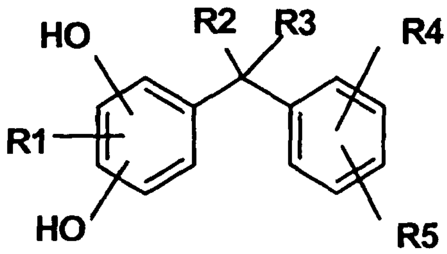

**1**

wobei gilt:

R1 ist

- Wasserstoff,
- Methyl,
- geradkettiges oder verzweigtes, gesättigtes oder ungesättigtes Alkyl mit 2-4 C-Atomen
- OH oder
- Halogen,

R2 ist

- Wasserstoff,
- Methyl oder
- geradkettiges oder verzweigtes, gesättigtes oder ungesättigtes Alkyl mit 2-5 C-Atomen,

R3 ist

- Methyl oder
- geradkettiges oder verzweigtes, gesättigtes oder ungesättigtes Alkyl mit 2-5 C-Atomen,

und
R4 und R5 sind unabhängig voneinander

- Wasserstoff,
- Methyl,
- geradkettiges oder verzweigtes, gesättigtes oder ungesättigtes Alkyl mit 2-5 C-Atomen
- OH oder
- Halogen

zur Herstellung eines Mittels zur Hautaufhellung und/oder zur Bekämpfung von Altersflecken.

**3.** Verwendung von Styrylresorcinolgemäß Anspruch 1 oder 2.

**4.** Duftstoffkomposition, umfassend

(a) eine sensorisch wirksame Menge eines Duftstoffes,
(b) eine Tyrosinase-inhibierend wirkende Menge einer oder mehrerer Verbindungen der Formel 1

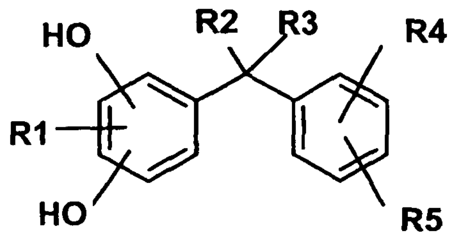

**1**

wobei gilt:

R1 ist

- Wasserstoff,
- Methyl,
- geradkettiges oder verzweigtes, gesättigtes oder ungesättigtes Alkyl mit 2-4 C-Atomen
- OH oder
- Halogen,

R2 ist

- Wasserstoff,
- Methyl oder
- geradkettiges oder verzweigtes, gesättigtes oder ungesättigtes Alkyl mit 2-5 C-Atomen,

R3 ist

- Methyl oder
- geradkettiges oder verzweigtes, gesättigtes oder ungesättigtes Alkyl mit 2-5 C-Atomen,

und
R4 und R5 sind unabhängig voneinander

- Wasserstoff,
- Methyl,
- geradkettiges oder verzweigtes, gesättigtes oder ungesättigtes Alkyl mit 2-5 C-Atomen
- OH oder
- Halogen.

**5.** Kosmetische Zubereitung, umfassend

- ein oder mehrere Verbindungen zur Pflege und/oder Reinigung von (a) Haut und/oder (b) Haaren sowie
- eine Tyrosinase-inhibierend wirkende Menge einer oder mehrerer
- Verbindungen der Formel 1

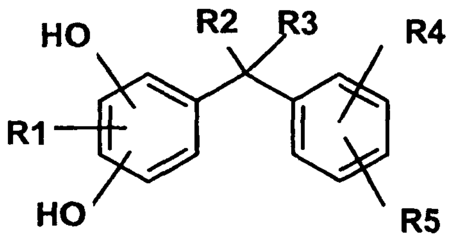

**1**

wobei gilt:

R1 ist

- Wasserstoff,
- Methyl,
- geradkettiges oder verzweigtes, gesättigtes oder ungesättigtes Alkyl mit 2-4 C-Atomen
- OH oder
- Halogen,

R2 ist

- Wasserstoff,
- Methyl oder
- geradkettiges oder verzweigtes, gesättigtes oder ungesättigtes Alkyl mit 2-5 C-Atomen,

R3 ist
- Methyl oder
- geradkettiges oder verzweigtes, gesättigtes oder ungesättigtes Alkyl mit 2-5 C-Atomen,
und
R4 und R5 sind unabhängig voneinander

- Wasserstoff,
- Methyl,
- geradkettiges oder verzweigtes, gesättigtes oder ungesättigtes Alkyl mit 2-5 C-Atomen
- OH oder
- Halogen.

**6.** Pharmazeutisches Produkt, umfassend

- ein oder mehrere Verbindungen zur Pflege und/oder Reinigung von (a) Haut und/oder (b) Haaren sowie
- eine Tyrosinase-inhibierend wirkende Menge einer oder mehrerer
- Verbindungen der Formel 1

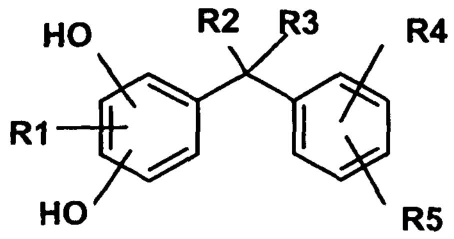

**1**

wobei gilt:

R1 ist

- Wasserstoff,
- Methyl,
- geradkettiges oder verzweigtes, gesättigtes oder ungesättigtes Alkyl mit 2-4 C-Atomen
- OH oder
- Halogen,

R2 ist

- Wasserstoff,
- Methyl oder
- geradkettiges oder verzweigtes, gesättigtes oder ungesättigtes Alkyl mit 2-5 C-Atomen,

R3 ist

- Methyl oder
- geradkettiges oder verzweigtes, gesättigtes oder ungesättigtes Alkyl mit 2-5 C-Atomen,

und
R4 und R5 sind unabhängig voneinander

- Wasserstoff,
- Methyl,
- geradkettiges oder verzweigtes, gesättigtes oder ungesättigtes Alkyl mit 2-5 C-Atomen
- OH oder
- Halogen.

**7.** Sonnenschutz-Zubereitung, umfassend

- eine wirksame Menge eines UV-Filters, so dass der Lichtschutzfaktor der Sonnenschutz-Zubereitung > 2 ist, sowie
- eine Tyrosinase-inhibierend wirkende Menge einer oder mehrerer
- Verbindungen der Formel 1

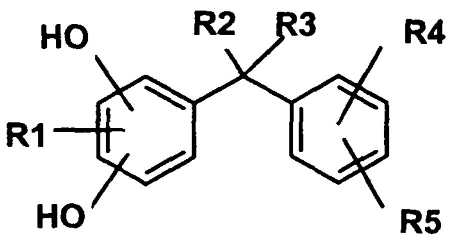

**1**

wobei gilt:

R1 ist

- Wasserstoff,
- Methyl,
- geradkettiges oder verzweigtes, gesättigtes oder ungesättigtes Alkyl mit 2-4 C-Atomen
- OH oder
- Halogen,

R2 ist

- Wasserstoff,
- Methyl oder
- geradkettiges oder verzweigtes, gesättigtes oder ungesättigtes Alkyl mit 2-5 C-Atomen,

R3 ist

- Methyl oder
- geradkettiges oder verzweigtes, gesättigtes oder ungesättigtes Alkyl mit 2-5 C-Atomen,

und
R4 und R5 sind unabhängig voneinander

- Wasserstoff,
- Methyl,
- geradkettiges oder verzweigtes, gesättigtes oder ungesättigtes Alkyl mit 2-5 C-Atomen
- OH oder
- Halogen.

**8.** Verwendung einer Verbindung der Formel 1 oder einer Substanzmischung, die eine oder mehrere Verbindungen der Formel 1 umfasst,

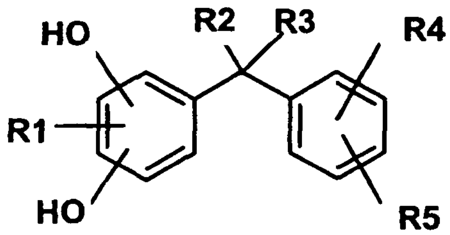

**1**

wobei gilt:

R1 ist

- Wasserstoff,
- Methyl,
- geradkettiges oder verzweigtes, gesättigtes oder ungesättigtes Alkyl mit 2-4 C-Atomen
- OH oder
- Halogen,

R2 ist

- Wasserstoff,
- Methyl oder
- geradkettiges oder verzweigtes, gesättigtes oder ungesättigtes Alkyl mit 2-5 C-Atomen,

R3 ist

- Methyl oder
- geradkettiges oder verzweigtes, gesättigtes oder ungesättigtes Alkyl mit 2-5 C-Atomen,

und
R4 und R5 sind unabhängig voneinander

- Wasserstoff,
- Methyl,
- geradkettiges oder verzweigtes, gesättigtes oder ungesättigtes Alkyl mit 2-5 C-Atomen
- OH oder
- Halogen

zur Herstellung eines Mittels (a) gegen Haut- und Haarbräunung, (b) zur Bekämpfung von Altersflecken und/oder (c) zur Inhibierung der unerwünschten Bräunung von Lebensmitteln.

## Claims

**1.** Use of a compound of the formula 1 or of a substance mixture which comprises one or more compounds of the formula 1

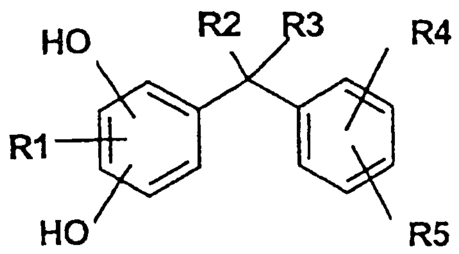

**1**

wherein:

R1 is

- hydrogen,
- methyl,
- straight-chain or branched, saturated or unsaturated alkyl having 2-4 C atoms,
- OH or
- halogen,

R2 is

- hydrogen,
- methyl or
- straight-chain or branched, saturated or unsaturated alkyl having 2-5 C atoms,

R3 is

- methyl or
- straight-chain or branched, saturated or unsaturated alkyl having 2-5 C atoms,

and
R4 and R5 are, independently of one another,

- hydrogen,
- methyl,
- straight-chain or branched, saturated or unsaturated alkyl having 2-5 C atoms,
- OH or
- halogen

as a tyrosinase inhibitor for lightening hair and/or as a browning inhibitor in the foodstuffs industry.

**2.** Use of a compound of the formula 1 or of a substance mixture which comprises one or more compounds of the formula 1

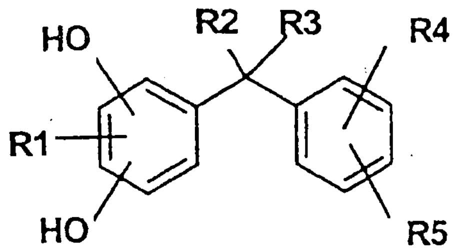

1

wherein:

R1 is

- hydrogen,
- methyl,
- straight-chain or branched, saturated or unsaturated alkyl having 2-4 C atoms,
- OH or
- halogen,

R2 is

- hydrogen,
- methyl or
- straight-chain or branched, saturated or unsaturated alkyl having 2-5 C atoms,

R3 is

- methyl or
- straight-chain or branched, saturated or unsaturated alkyl having 2-5 C atoms,

and
R4 and R5 are, independently of one another,

- hydrogen,
- methyl,
- straight-chain or branched, saturated or unsaturated alkyl having 2-5 C atoms,
- OH or
- halogen

for the preparation of a composition for lightening the skin and/or for combating age spots.

**3.** Use of styrylresorcinol according to claim 1 or 2.

**4.** Fragrance composition comprising

(a) a sensorially active amount of a fragrance
(b) one or more compounds, in an amount having a tyrosinase-inhibiting action, of the formula 1

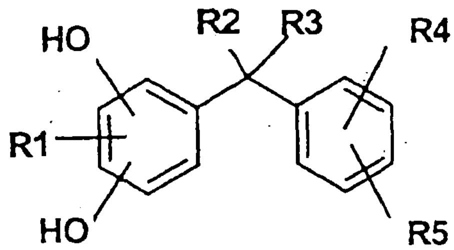

1

wherein:

R1 is

- hydrogen,
- methyl,
- straight-chain or branched, saturated or unsaturated alkyl having 2-4 C atoms,
- OH or
- halogen,

R2 is

- hydrogen,
- methyl or
- straight-chain or branched, saturated or unsaturated alkyl having 2-5 C atoms,

R3 is

- methyl or
- straight-chain or branched, saturated or unsaturated alkyl having 2-5 C atoms,

and
R4 and R5 are, independently of one another,

- hydrogen,
- methyl,
- straight-chain or branched, saturated or unsaturated alkyl having 2-5 C atoms,
- OH or
- halogen.

**5.** Cosmetic formulation, comprising

- one or more compounds for care and/or cleansing of (a) skin and/or (b) hair and
- one or more compounds, in an amount having a tyrosinase- inhibiting action, of the formula 1

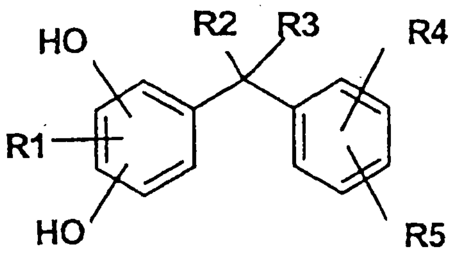

1

wherein:

R1 is

- hydrogen,
- methyl,
- straight-chain or branched, saturated or unsaturated alkyl having 2-4 C atoms,
- OH or
- halogen,

R2 is

- hydrogen,
- methyl or
- straight-chain or branched, saturated or unsaturated alkyl having 2-5 C atoms,

R3 is

- methyl or
- straight-chain or branched, saturated or unsaturated alkyl having 2-5 C atoms,

and
R4 and R5 are, independently of one another,

- hydrogen,
- methyl,
- straight-chain or branched, saturated or unsaturated alkyl having 2-5 C atoms,
- OH or
- halogen.

**6.** Pharmaceutical product, comprising

- one or more compounds for care and/or cleansing of (a) skin and/or (b) hair and
- one or more compounds, in an amount having a tyrosinase-inhibiting action, of the formula 1

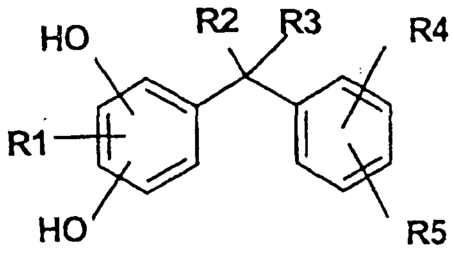

1

wherein:

R1 is

- hydrogen,
- methyl,
- straight-chain or branched, saturated or unsaturated alkyl having 2-4 C atoms,
- OH or
- halogen,

R2 is

- hydrogen,
- methyl or
- straight-chain or branched, saturated or unsaturated alkyl having 2-5 C atoms,

R3 is

- methyl or
- straight-chain or branched, saturated or unsaturated alkyl having 2-5 C atoms,

and
R4 and R5 are, independently of one another,

- hydrogen,
- methyl,
- straight-chain or branched, saturated or unsaturated alkyl having 2-5 C atoms,
- OH or
- halogen.

**7.** Sunscreen formulation, comprising

- an active amount of a UV filter, such that the light protection factor of the sunscreen formulation is > 2, and
- one or more compounds, in an amount having a tyrosinase-inhibiting action, of the formula 1

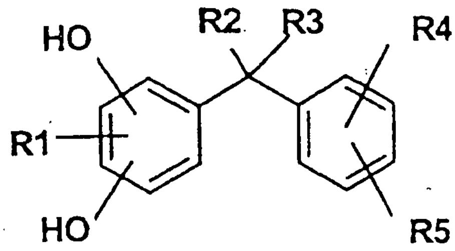

**1**

wherein:

R1 is

- hydrogen,
- methyl,
- straight-chain or branched, saturated or unsaturated alkyl having 2-4 C atoms,
- OH or
- halogen,

R2 is

- hydrogen,
- methyl or
- straight-chain or branched, saturated or unsaturated alkyl having 2-5 C atoms,

R3 is

- methyl or
- straight-chain or branched, saturated or unsaturated alkyl having 2-5 C atoms,

and
R4 and R5 are, independently of one another,

- hydrogen,
- methyl,
- straight-chain or branched, saturated or unsaturated alkyl having 2-5 C atoms,
- OH or
- halogen.

**8.** Use of a compound of the formula 1 or of a substance mixture which comprises one or more compounds of the formula 1

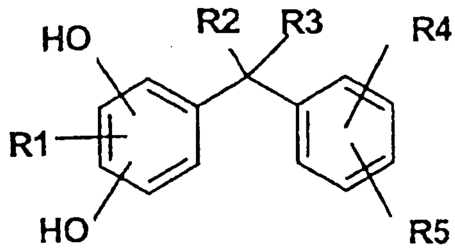

**1**

wherein:

R1 is

- hydrogen,
- methyl,
- straight-chain or branched, saturated or unsaturated alkyl having 2-4 C atoms,
- OH or
- halogen,

R2 is

- hydrogen,
- methyl or
- straight-chain or branched, saturated or unsaturated alkyl having 2-5 C atoms,

R3 is

- methyl or
- straight-chain or branched, saturated or unsaturated alkyl having 2-5 C atoms,

and
R4 and R5 are, independently of one another,

- hydrogen,
- methyl,
- straight-chain or branched, saturated or unsaturated alkyl having 2-5 C atoms,
- OH or
- halogen

for the preparation of a composition (a) against browning of skin and hair, (b) for combating age spots and/or (c) for inhibiting undesirable browning of foodstuffs.

## Revendications

**1.** Utilisation d'un composé de formule 1 ou d'un mélange de substances comprenant un ou plusieurs composés de formule 1

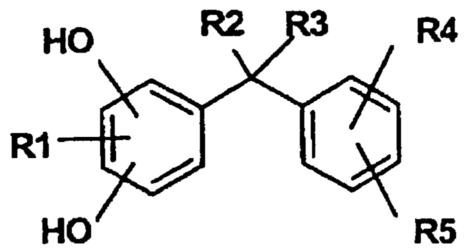

**1**

dans laquelle
R1 est

- hydrogène,
- méthyle,
- alkyle de 2-4 atomes de carbone linéaire ou ramifié, saturé ou insaturé,
- OH ou
- halogène,

R2 est

- hydrogène,
- méthyle ou
- alkyle de 2-5 atomes de carbone linéaire ou ramifié, saturé ou insaturé,

R3 est

- méthyle ou
- alkyle de 2-5 atomes de carbone linéaire ou ramifié, saturé ou insaturé,

et
R4 et R5 sont, indépendamment l'un de l'autre,

- hydrogène,
- méthyle,
- alkyle de 2-5 atomes de carbone linéaire ou ramifié, saturé ou insaturé,
- OH ou
- halogène,

comme inhibiteur de tyrosinase pour l'éclaircissement des cheveux et/ou comme inhibiteur du brunissement dans l'industrie alimentaire.

**2.** Utilisation d'un composé de formule 1 ou d'un mélange de substances comprenant un ou plusieurs composés de formule 1

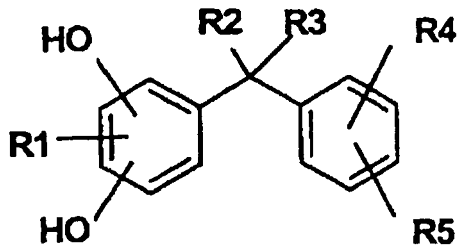

1

dans laquelle
R1 est

- hydrogène,
- méthyle,
- alkyle de 2-4 atomes de carbone linéaire ou ramifié, saturé ou insaturé,
- OH ou
- halogène,

R2 est

- hydrogène,
- méthyle ou
- alkyle de 2-5 atomes de carbone linéaire ou ramifié, saturé ou insaturé,

R3 est

- méthyle ou
- alkyle de 2-5 atomes de carbone linéaire ou ramifié, saturé ou insaturé,

et
R4 et R5 sont, indépendamment l'un de l'autre,

- hydrogène,
- méthyle,
- alkyle de 2-5 atomes de carbone linéaire ou ramifié, saturé ou insaturé,
- OH ou
- halogène,

pour la préparation d'un produit destiné à l'éclaircissement de la peau et/ou à la lutte contre les taches de vieillesse.

**3.** Utilisation de styrylrésorcinol selon la revendication 1 ou 2.

**4.** Composition de parfum, comprenant

(a) une quantité sensoriellement active d'un parfum,
(b) une quantité efficace inhibant la tyrosinase d'un ou plusieurs composés de formule 1

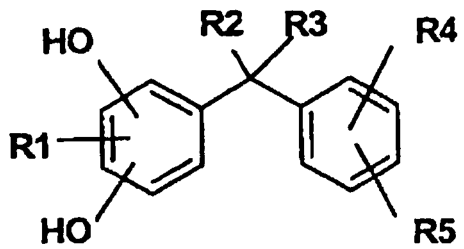

**1**

dans laquelle
R1 est

- hydrogène,
- méthyle,
- alkyle de 2-4 atomes de carbone linéaire ou ramifié, saturé ou insaturé,
- OH ou
- halogène,

R2 est

- hydrogène,
- méthyle ou
- alkyle de 2-5 atomes de carbone linéaire ou ramifié, saturé ou insaturé,

R3 est

- méthyle ou
- alkyle de 2-5 atomes de carbone linéaire ou ramifié, saturé ou insaturé,

et
R4 et R5 sont, indépendamment l'un de l'autre,

- hydrogène,
- méthyle,
- alkyle de 2-5 atomes de carbone linéaire ou ramifié, saturé ou insaturé,
- OH ou
- halogène.

**5.** Composition cosmétique, comprenant

- un ou plusieurs composés pour les soins et/ou le nettoyage (a) de la peau et/ou (b) des cheveux, ainsi que
- une quantité efficace inhibant la tyrosinase d'un ou plusieurs composés de formule 1

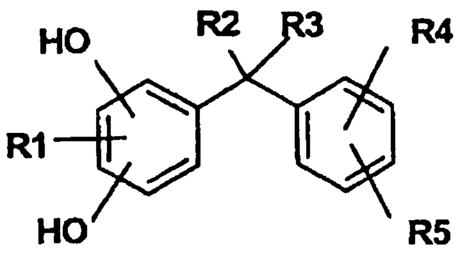

1

dans laquelle
R1 est

- hydrogène,
- méthyle,
- alkyle de 2-4 atomes de carbone linéaire ou ramifié, saturé ou insaturé,
- OH ou
- halogène,

R2 est

- hydrogène,
- méthyle ou
- alkyle de 2-5 atomes de carbone linéaire ou ramifié, saturé ou insaturé,

R3 est

- méthyle ou
- alkyle de 2-5 atomes de carbone linéaire ou ramifié, saturé ou insaturé,

et
R4 et R5 sont, indépendamment l'un de l'autre,

- hydrogène,
- méthyle,
- alkyle de 2-5 atomes de carbone linéaire ou ramifié, saturé ou insaturé,
- OH ou
- halogène.

**6.** Produit pharmaceutique, comprenant

- un ou plusieurs composés pour les soins et/ou le nettoyage (a) de la peau et/ou (b) des cheveux, ainsi que
- une quantité efficace inhibant la tyrosinase d'un ou plusieurs composés de formule 1

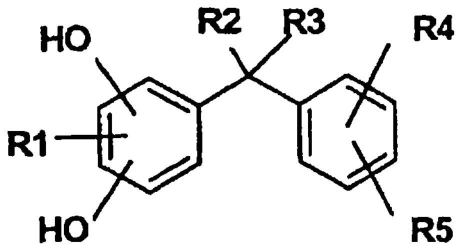

**1**

dans laquelle
R1 est

- hydrogène,
- méthyle,
- alkyle de 2-4 atomes de carbone linéaire ou ramifié, saturé ou insaturé,
- OH ou
- halogène,

R2 est

- hydrogène,
- méthyle ou
- alkyle de 2-5 atomes de carbone linéaire ou ramifié, saturé ou insaturé,

R3 est

- méthyle ou
- alkyle de 2-5 atomes de carbone linéaire ou ramifié, saturé ou insaturé,

et
R4 et R5 sont, indépendamment l'un de l'autre,

- hydrogène,
- méthyle,
- alkyle de 2-5 atomes de carbone linéaire ou ramifié, saturé ou insaturé,
- OH ou
- halogène.

**7.** Composition solaire, comprenant

- une quantité active d'un filtre UV, de façon que le facteur de protection contre la lumière de la composition solaire soit > 2, ainsi que
- une quantité efficace inhibant la tyrosinase d'un ou plusieurs composés de formule 1

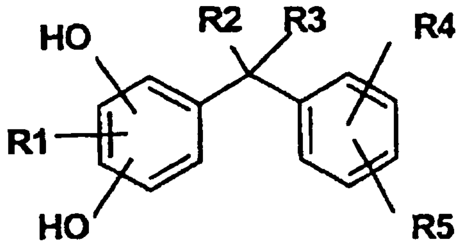

**1**

dans laquelle
R1 est

- hydrogène,
- méthyle,
- alkyle de 2-4 atomes de carbone linéaire ou ramifié, saturé ou insaturé,
- OH ou
- halogène,

R2 est

- hydrogène,
- méthyle ou
- alkyle de 2-5 atomes de carbone linéaire ou ramifié, saturé ou insaturé,

R3 est

- méthyle ou
- alkyle de 2-5 atomes de carbone linéaire ou ramifié, saturé ou insaturé,

et
R4 et R5 sont, indépendamment l'un de l'autre,

- hydrogène,
- méthyle,
- alkyle de 2-5 atomes de carbone linéaire ou ramifié, saturé ou insaturé,
- OH ou
- halogène.

**8.** Utilisation d'un composé de formule 1 ou d'un mélange de substances comprenant un ou plusieurs composés de formule 1

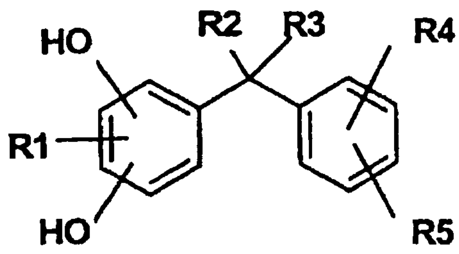

**1**

dans laquelle
R1 est

- hydrogène,
- méthyle,
- alkyle de 2-4 atomes de carbone linéaire ou ramifié, saturé ou insaturé,
- OH ou
- halogène,

R2 est

- hydrogène,
- méthyle ou
- alkyle de 2-5 atomes de carbone linéaire ou ramifié, saturé ou insaturé,

R3 est

- méthyle ou
- alkyle de 2-5 atomes de carbone linéaire ou ramifié, saturé ou insaturé,

et
R4 et R5 sont, indépendamment l'un de l'autre,

- hydrogène,
- méthyle,
- alkyle de 2-5 atomes de carbone linéaire ou ramifié, saturé ou insaturé,
- OH ou
- halogène,

pour la préparation d'un produit (a) contre le brunissement de la peau et des cheveux, (b) pour lutter contre les taches de vieillesse et/ou (c) pour inhiber le brunissement indésirable des aliments.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5399785 A
- WO 0056279 A
- EP 0811595 A1


### In der Beschreibung aufgeführte Nicht-Patentliteratur


- *Cosmetics & Toiletries,* 1996, vol. 111 (5), 43-51
- **T. YAMAMURA et al.** *Bull. Chem. Soc. Jpn.,* 1995, vol. 68, 2955-2960